(19) Europäisches Patentamt
European Patent Office
Office européen des brevets

(11) Numéro de publication: **0 228 942 B1**

(12) **FASCICULE DE BREVET EUROPEEN**

(45) Date de publication du fascicule du brevet: **23.05.90**

(51) Int. Cl.[5]: **C07C 69/743**, C07C 255/49, C07D 213/64, A01N 53/00, A23K 1/00

(21) Numéro de dépôt: **86402701.6**

(22) Date de dépôt: **05.12.86**

(54) **Nouveaux dérivés de l'acide cyclopropane carboxylique à substituant iodé, leur préparation, leur application à la lutte contre les parasites des végétaux et des animaux et les compositions les renfermant.**

(30) Priorité: **10.12.85 FR 8518226**
**09.07.86 FR 8609997**

(43) Date de publication de la demande:
**15.07.87 Bulletin 87/29**

(45) Mention de la délivrance du brevet:
**23.05.90 Bulletin 90/21**

(84) Etats contractants désignés:
**AT BE CH DE ES FR GB IT LI LU NL**

(56) Documents cités:
**EP-A- 0 000 229**
**EP-A- 0 050 093**
**DE-A- 2 941 332**
**GB-A- 2 058 784**

**J.Chem. Soc. Commun. (1985) January**

(73) Titulaire: **ROUSSEL-UCLAF, 35, boulevard des Invalides, F-75007 Paris(FR)**

(72) Inventeur: **Tessier, Jean, 30, rue Jean Moulin, F-94300 Vincennes(FR)**
Inventeur: **Demoute, Jean-Pierre, 249 bis, rue de Rosny, F-93100 Montreuil Sous Bois(FR)**
Inventeur: **Cadiergue, Joseph, 6, rue Jules Princet, F-93600 Aulnay Sous Bois(FR)**

(74) Mandataire: **Tonnellier, Marie-José, Département des Brevets ROUSSEL UCLAF B.P. no 9, F-93230 Romainville(FR)**

**EP 0 228 942 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne de nouveaux dérivés de l'acide cyclopropane carboxylique à substituant iodé, leur préparation, leur application à la lutte contre les parasites des végétaux et des animaux et les compositions les renfermant.

L'invention a pour objet les composés de formule générale (I) sous toutes leurs formes stéréoisomères ou sous forme de mélanges de stéréoisomères :

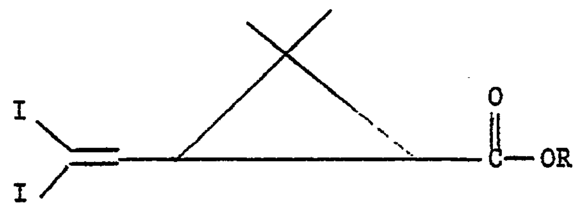

(I)

dans laquelle R représente le reste d'un alcool utilisé dans le domaine des pyrétrinoïdes.

On connaissait des dérivés chlorés ou bromés correspondant aux produits de formule générale I qui étaient décrits dans le brevet français 2 185 612.

La société demanderesse a maintenant découvert que les produits iodés de formule générale (I) sont doués de remarquables propriétés pesticides.

L'art antérieur n'incitait pas le chimiste à préparer des dérivés diiodovinyliques, la matière première mise en œuvre, le tétraiodure de carbone est peu stable, c'est donc un réactif difficile à manier. On pouvait, de plus, également s'attendre à ce que les produits finals obtenus soient trop instables pour pouvoir être utilisés en agriculture. Il n'en est rien. On vient de découvrir que les produits diiodovinyliques de formule I pouvaient être préparés, qu'ils étaient stables et doués d'exellentes propriétés pesticides.

La réaction de Wittig décrite pour le tétrachlorure de carbone ou le tétrabromure de carbone dans le brevet français 2 185 612 était inapplicable telle quelle aux dérivés tétraiodés.

Les 1,1-diiodoalcènes étaient en effet jusqu'à une époque trés récente une classe de composés à peu prés inconnue, puisque seul le diodométhylène cyclohexane avait été préparé à l'aide d'intermédiaire du bore (F. Gaviña et coll J. Chem. Soc. Chem. Commun. p. 296 (1985)) F. Gaviña et coll ont réalisé pour la première fois la transformation des aldéhydes aliphatiques et aromatiques par une réaction de Wittig en 1,1-diiodo alcènes en utilisant la triphényl phosphine et le tétraiodure de carbone. La réaction est très délicate et ne marche qu'avec du tétraiodure de carbon trés pur en opérant aux environs de 0°C.

La société demanderesse a maintenant découvert que la réaction de F. Gaviña et coll était applicable aux aldéhydes 3-formyl 2,2-diméthyl cyclopropane carboxyliques pour fournir les acides 2,2-diméthyl 3(2,2-diiodoéthényl) cyclopropane carboxyliques.

L'invention a notamment pour object les composés de formule générale (I) sous toutes leurs formes stéréoisomères ou sous forme de mélanges de stéréoisomères, caractérisés en ce que R représente:

– soit un radical alcoyle, linéaire ou ramifié, contenant de 1 à 8 atomes de carbone,

– soit un radical benzyle,

– soit un groupement de formule:

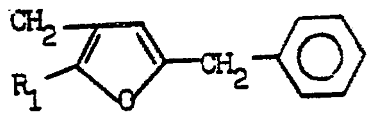

dans laquelle $R_1$ représente un atome d'hydrogène ou un radical méthyle,

– soit un groupement de formule:

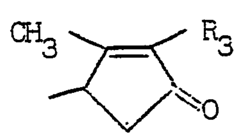

dans laquelle $R_3$ représente un radical aliphatique organique contenant de 2 à 6 atomes de carbone et une ou plusieurs insaturations carbone-carbone et plus particulièrement les radicaux :

$-CH_2-CH = CH_2$,

$-CH_2-CH = CH-CH_3$,

$-CH_2-CH = CH-CH_2-CH_3$,

$-CH_2-CH = CH-CH = CH_2$,

$-CH_2-C\equiv CH$,

- soit un groupement de formule :

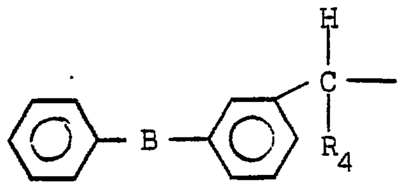

dans laquelle B représente un groupement, C = O ou un atome d'oxygène et $R_4$ représente un atome d'hydrogène, un radical méthyle, un groupement -C≡N, un groupement -C≡CH, un groupement

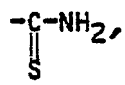

- soit un groupement de formule :

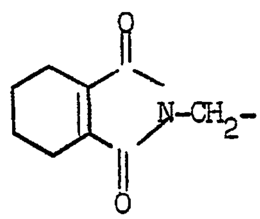

- soit un groupement de formule :

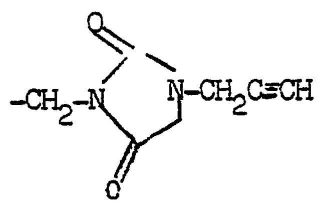

- soit un groupement de formule :

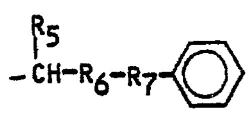

dans laquelle $R_5$ représente un atome d'hydrogène ou un radical -CN,
$R_6$ représente un radical thiazolyle dans lequel la liaison

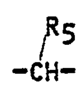

peut se trouver à l'une quelconque des positions disponibles,
$R_7$ représente un radical $-CH_2-$ ou un atome d'oxygène :
- soit un groupement de formule :

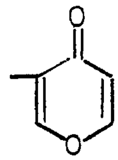

- soit un groupement de formule :

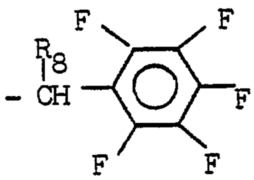

dans laquelle $R_8$ représente un atome d'hydrogène ou un radical CN,
- soit un groupement de formule :

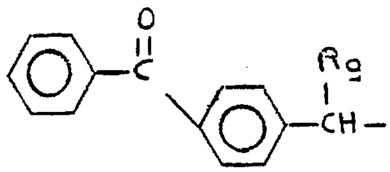

dans laquelle $R_9$ représente un atome d'hydrogène ou radical -CN
- soit un groupement de formule :

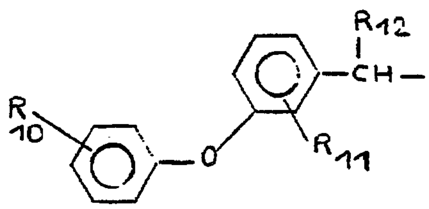

dans laquelle $R_{12}$ représente un atome d'hydrogène, un radical méthyle, éthynyle ou cyano et $R_{10}$ et $R_{11}$ différents l'un de l'autre représentent un atome d'hydrogène, de fluor, de chlore ou de brome ;
- soit un groupement de formule :

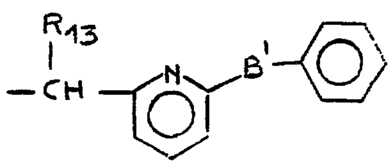

dans laquelle $R_{13}$ représente un atome d'hydrogène, un radical méthyle, éthynyle ou cyano et B′ représente un atome d'oxygène ou de soufre,
- soit un groupement de formule :

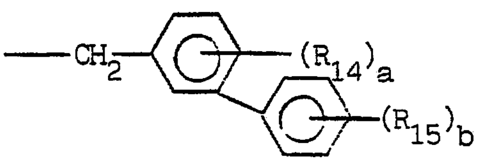

dans laquelle $R_{14}$ représente un atome d'halogène, un radical alcoyle renfermant de 1 à 6 atomes de carbone ou un radical trifluorométhyle, $R_{15}$ représente un atome d'halogène, un radical alcoyle renfermant de 1 à 6 atomes de carbone, un radical alcoxy renfermant de 1 à 6 atomes de carbone ou un radical trifluorométhyle, a est 0,1, 2, 3 ou 4 et b est 0, 1, 2, 3, 4 ou 5.

Dans les composés selon l'invention, lorsque R représente un radical alcoyle, linéaire ou ramifié, contenant de 1 à 8 atomes de carbone, il peut représenter notamment un radical méthyle, éthyle, propyle linéaire ou ramifié, butyle linéaire ou ramifié, pentyle linéaire ou ramifié, hexyle linéaire ou ramifié, octyle linéaire ou ramifié.

L'invention a plus spécialement pour objet les composés de formule générale (I) sous toutes leurs formes stéréoisomères ou sous forme de mélanges de stéréoisomères, caractérisés en ce que R représente un groupement de formule :

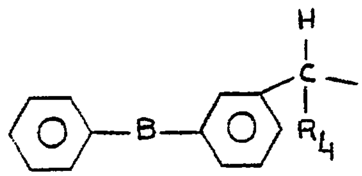

dans laquelle B représente un groupement C = 0 ou un atome d'oxygène et $R_4$ représente un atome d'hydrogène, un radical méthyle, un groupement -C≡N, un groupement -C ≡ CH, ou un groupement

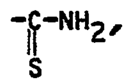

et notamment le groupement:

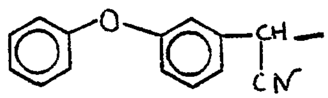

,

les composés de formule générale (I) caractérisés en ce que R représente un groupement de formule :

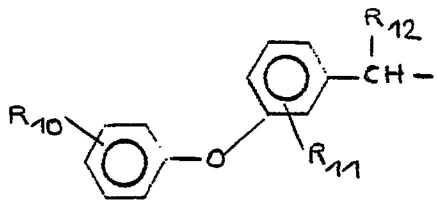

dans laquelle $R_{12}$ représente un atome d'hydrogène, un radical méthyle, éthynyle ou cyano et $R_{10}$ et $R_{11}$ différents l'un de l'autre représentent un atome d'hydrogène, de fluor, de chlore ou de brome, et notamment le groupement de formule :

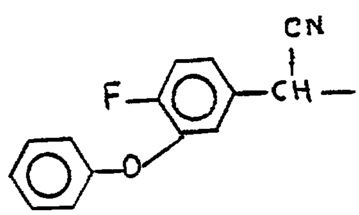

les composés de formule générale (I) caractérisés en ce que R représente un groupement de formule :

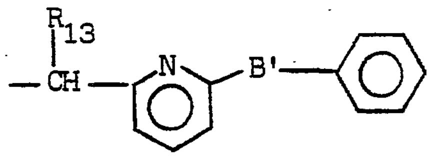

dans laquelle $R_{13}$ représente un atome d'hydrogène, un radical méthyle, éthynyle ou cyano et B' représente un atome d'oxygène ou de soufre, et notamment le groupement :

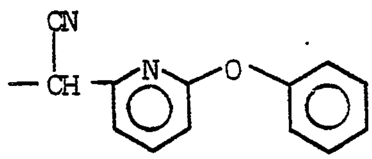

les composés de formule générale (I) caractérisés en ce que R représente un groupement de formule :

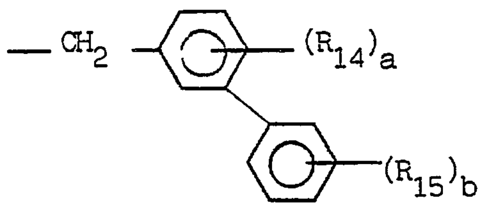

dans laquelle $R_{14}$ représente un atome d'halogène, un radical alcoyle renfermant de 1 à 6 atomes de carbone ou un radical trifluorométhyle, $R_{15}$ représente un atome d'halogène, un radical alcoyle renfermant de 1 à 6 atomes de carbone, un radical alcoxy renfermant de 1 à 6 atomes de carbone ou un radical trifluorométhyle, a est 0, 1, 2, 3 ou 4 et b est 0, 1, 2, 3, 4 ou 5 et notamment le groupement :

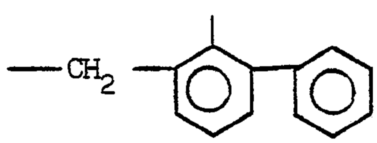

L'invention a plus particulièrement pour objet les composés dont les noms suivent :
- le 1R, cis 2,2-diméthyl 3-/2,2-diiodo éthényl/cyclopropane carboxylate de (S) cyano (3-phénoxy 4-fluoro phényl) méthyle,
- le 1R, cis 2,2-diméthyl 3-(2,2-diiodoéthényl) cyclopropane carboxylate de (S) cyano 1-(3-phénoxy 4-fluorophényl) méthyle et le 1S, cis 2,2-diméthyl 3-(2,2-diiodoéthényl) cyclopropane carboxylate de (R) cyano 1-(3-phénoxy 4-fluorophényl) méthyle en mélange 50 - 50.

L'invention a pour objet un procédé de préparation des composés de formule générale (I) sous toutes leurs formes stéréoisomères ou sous forme de mélanges de stéréoisomères, caractérisé en que l'on fait réagir le tétraiodométhane et la triphénylphosphine, au sein d'un solvant organique avec un composé de formule (II) :

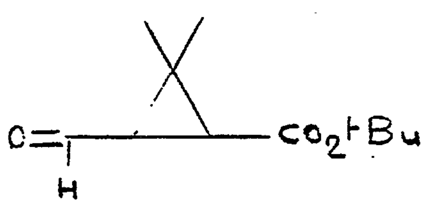

(II)

sous toutes ses formes stéréoisomères ou sous forme de mélanges de stéréoisomères, soumet le composé résultantde formule (III) :

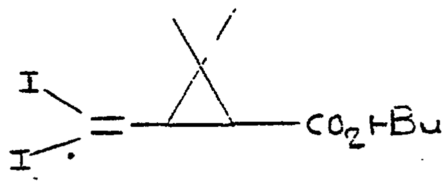

(III)

à l'action de l'acide paratoluène sulfonique au sein d'un solvant organique pour obtenir un composé de formule (IV) :

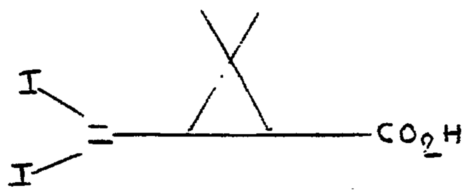

(IV)

que l'on transforme éventuellement en dérivé fonctionnel tel que chlorure d'acide, anhydride ou anhydride mixte, fait réagir l'acide de formule (IV) ou le dérivé fonctionnel de cet acide sur l'alcool de formule :
R-OH
ou sur un dérivé fonctionnel de cet alcool pour obtenir le produit de formule générale (I) correspondant :

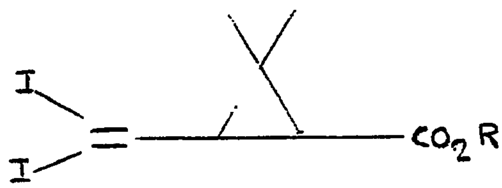

(I)

Dans un mode opératoire préféré du procédé de l'invention :
- le solvant organique au sein duquel on fait réagir le tétraiodométhane et la triphénylphosphine avec un composé de formule (II) est un solvant aliphatique chloré, tel que le chlorure de méthylène,
- le solvant organique au sein duquel ont fait réagir l'acide paratoluène sulfonique sur un composé de formule (III) est un solvant aromatique tel que le benzène, le toluène ou le xylène,
- le dérivé fonctionnel de l'acide est le chlorure d'acide et l'on fait réagir le chlorure d'acide sur l'alcool R-OH en présence d'une base tertiaire,
- on fait réagir l'acide de formule (IV) avec l'alcool ROH, en présence de dicyclohexyl carbodiimide ou de diisopropyl carbodiimide, et de diméthyl amino pyridine.

L'invention a également pour objet un procédé de préparation des composés de formule générale (I) selon le procédé tel que défini ci-dessus, caractérisé en ce que l'on fait réagir le composé de formule (II):

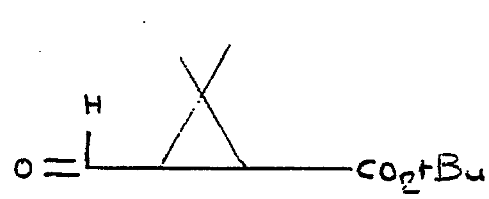

(II)

avec le tétraiodométhane, la triphénylphosphine et le zinc au sein d'un solvant organique pour obtenir un composé de formule (IV) :

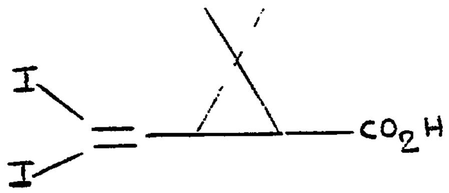

(IV)

puis termine la synthèse comme ci-dessus.

Dans un mode opératoire préféré du procédé ci-dessus décrit :
le solvant organique au sein duquel on fait réagir le tétraiodométhane, la triphénylphosphine et le zinc avec un composé de formule (II), est un solvant aliphatique chloré, tel que le chlorure de méthylène.

Les composés de formule I présentent d'intéressantes propriétés qui permettent leur utilisation dans la lutte contre les parasites. Il peut s'agir, par exemple, de la lutte contre les parasites des végétaux, les parasites des locaux et les parasites des animaux à sang chaud.

C'est ainsi que l'on peut utiliser les produits de formule I pour lutter contre les insectes, les nématodes et les acariens parasites des végétaux et des animaux.

L'invention a donc pour objet les composés de formule (I) pour leur utilisation à la lutte contre les parasites des végétaux, les parasites des locaux et les parasites des animaux à sang chaud.

L'invention a donc également pour objet les compositions pesticides destinées à la lutte contre les parasites des végétaux, les parasites des locaux et les parasites des animaux à sang chaud, caractérisées en ce qu'elles renferment comme principe actif, au moins un composé défini précédemment.

Dans les compositions destinées à l'usage agricole et à l'usage dans les locaux, la ou les matières actives peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employés classiquement pour l'utilisation de ce genre de composés.

Outre le principe actif, ces compositions contiennent, en général, un véhicule et/ou un agent tensioactif, non ionique assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les produits de formule (I) peuvent être utilisés notamment pour lutter contre les insectes dans le domaine agricole, pour lutter, par exemple, contre les pucerons, les larves de lépidoptères et les coléoptères. Ils sont utilisés à des doses comprises entre 10 g et 300 g de matière active à l'hectare.

Les produits de formule (I) peuvent également être utilisés pour lutter contre les insectes dans les locaux, pour lutter notamment contre les mouches, les moustiques et les blattes.

Les produits de formule (I) peuvent également être utilisés pour lutter contre les insectes parasites des animaux, par exemple, les poux notamment chez les bovins, les ovins et les volailles.

L'invention a donc notamment pour objet les compositions insecticides renfermant comme principe actif au moins l'un des composés définis précédemment.

L'invention a tout spécialement pour objet les compositions insecticides renfermant comme principe actif les produits dont les les noms suivent :
- le 1R,cis 2,2-diméthyl 3-/2,2-diiodo éthényl/cyclopropane carboxylate de (S) cyano (3-phénoxy 4-fluoro phényl) méthyle,
- le 1R,cis 2,2-diméthyl 3-(2,2-diiodoéthényl) cyclopropane carboxylate de (S) cyano 1-(3-phénoxy 4-fluorophényl) méthyle et le 1S,cis 2,2-diméthyl 3-(2,2-diiodoéthényl) cyclopropane carboxylate de (R) cyano 1-(3-phénoxy 4-fluorophényl) méthyle en mélange 50 - 50.

Les compositions insecticides selon l'invention contiennent de préférence de 0,005 % à 10 % en poids de matière active.

Selon un mode opératoire avantageux, pour un usage dans les locaux, les compositions insecticides selon l'invention sont utilisées sous forme de compositions fumigantes.

Les compositions insecticides selon l'invention peuvent alors être avantageusement constituées, pour la partie non active, d'un serpentin combustible ou encore d'un substrat fibreux incombustible. Dans ce dernier cas, le fumigant obtenu après incorporation de la matière active est placé sur un appareil chauffant tel qu'un électro-émanateur.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin) amidon et poudre de coque de noix de coco.

La dose de matière active peut alors être, par exemple, de 0,03 % à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

Les composés de l'invention peuvent également être utilisés pour lutter contre les acariens parasites des végétaux. La partie biologique exposée ci-après met clairement en évidence les remarquables propriétés acaricides du produit de l'exemple 4.

Les composés de l'invention peuvent également être utilisés pour lutter contre les nématodes parasites des végétaux.

L'invention a donc également pour objet les compositions acaricides ainsi que les compositions nématicides renfermant comme principe actif, au moins un composé de formule I.

Les compositions acaricides et nématicides peuvent se présenter notamment sous forme de poudres, granulés, suspensions, émulsions, solutions.

Pour l'usage acaricide, on utilise de préférence des poudres mouillables, pour pulvérisation foliaire, contenant de 1 à 80 % de principe actif ou des liquides pour pulvérisation foliaire contenant de 1 à 500 g/l de principe actif. On peut également employer des poudres pour poudrages foliaires contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Les composés de formule (I) peuvent encore être utilisés pour lutter contre les acariens parasites des animaux, pour lutter, par exemple, contre les tiques et notamment les tiques de l'espèce Boophilus, ceux de l'espèce Hyalomnia, ceux de l'espèce Amblyomnia et ceux de l'espèce Rhipicephalus ou pour lutter contre toutes sortes de gales et notamment la gale sarcoptique, la gale psoroptique et la gale chorioptique.

L'invention a donc également pour objet les compositions utilisées dans la lutte contre les acariens parasites des animaux à sang chaud, caractérisées en ce qu'elles renferment au moins un produit défini ci-dessus.

Ces compositions peuvent être administrées par voie externe, par vaporisation, par shampooing, par bain ou badigeonnage. Elles peuvent être également administrées par badigeonnage de l'épine dorsale selon la méthode dite méthode "pour on". Elles peuvent être également administrées par voie digestive.

Lorsqu'il s'agit de lutter contre les acariens parasites des animaux, on incorpore très souvent les produits de l'invention dans des compositions alimentaires en association avec un mélange nutritif adapté à l'alimentation animale. Le mélange nutritif peut varier selon l'espèce animale, il peut renfermer des céréales, des sucres et des grains, des tourteaux de soja, d'arachide et de tournesol, des farines d'origine animale, par exemple, des farines de poissons, des acides aminés de synthèse, des sels minéraux, des vitamines et des anti-oxydants.

L'invention a ainsi également pour objet les compositions destinées à l'alimentation animale renfermant comme principe actif au moins l'un des produits définis précédemment.

Pour exalter l'activité biologique des produits de l'invention, on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl)bicyclo/2,2,1/ 5-heptèn-2,3-dicarboximide, ou le pipéronyl-bis-2-(2'n-butoxy éthoxy) éthylacétal (ou tropital).

L'invention a également pour objet les associations douées d'activité insecticide, acaricide ou nématicide, caractérisées en ce qu'elles contiennent comme matière active, d'une part, un au moins des composés de formule générale (I), et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzylique des acides chrysanthémiques, par les esters d'alcool 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools α-cyano 3-phénoxy benzyliques des acides 2,2-diméthyl 3-(2,2-dichlorovinyl) cycloprane-1-carboxyliques, par les esters d'alcools α-cyano 3-phénoxy benzyliques d'acides 2,2-diméthyl 3-(2,2-dibromovinyl) cyclopropane-1-carboxyliques, par les esters d'alcool 3-phénoxy benzylique des acides 2-parachlophényl 2-isopropyl acétiques, par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzylique des acides 2,2-diméthyl 3-(1,2,2,2-tétrahaloéthyl) cyclopropane-1-carboxyliques, dans lesquels "halo" représente un atome de fluor, de chlore ou de brome, étant entendu que les composés I peuvent exister sous toutes leurs formes stéréoisomères possibles de même que les copules acides et alcools des esters pyréthrinoïdes ci-dessus.

Les associations, selon l'invention, présentent notamment l'intérêt soit de permettre de combattre par la polyvalence de leur action, une gamme de parasites, plus étendue, soit de manifester, dans certains cas, un effet de synergie.

Les exemples suivants illustrent l'invention sans toutefois la limiter.

Exemple 1 : 1R, trans 2,2-diméthyl 3-/2,2-diiodoéthényl/cyclopropane carboxylate de 1,1-diméthyl éthyle

On mélange 10,1 g de triphényl phosphine, 180 cm3 de chlorure de méthylène, introduit à 0°C 10 g de tétraiodométhane, agite pendant 20 minutes à 0°C, ajoute progressivement une solution de 1,9 g de 1R, trans 3-formyl 2,2-diméthyl cyclopropane carboxylate de terbutyle dans 20 cm3 de chlorure de méthylène, agite pendant 20 heures à +20°C, concentre à sec par distillation sous pression réduite, chromatographie le résidu sur silice en éluant par un mélange de chlorure de méthylène et d'acétate d'éthyle (1/1) puis par un mélange de chlorure de méthylène et d'hexane (3/7) et obtient 2,5 g de produit recherché.

Spectre IR (chloroforme) :

Absorption à 1 710cm-1 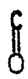 de l'ester terbutylique ;

1 370cm-1 $-CH_3$ ;
1 190cm-1 C-0-C

Spectre de RMN (deutérochloroforme) :

Pics à 1,2-1,3 ppm attribués aux hydrogènes des méthyles géminés.
Pic à 1,47 ppm attribué aux hydrogènes du terbutyle.
Pics à 1,55-1,65 ppm attribués à l'hydrogène en position 1 du cyclopropane.
Pics de 1,83 à 2,03 ppm attribués à l'hydrogène en position 3 du cyclopropane.
Pics à 6,75-6,87 ppm attribués à l'hydrogène de l'éthényle.

Exemple 2 : 1R, cis 2,2-diméthyl 3-/2,2-diiodo éthényl/ cyclopropane carboxylate de 1,1-diméthyl éthyle.

On mélange 10,1 g de triphényl phosphine, 180 cm3 de chlorure de méthylène, introduit à 0°C 10 g de tétraiodométhane, agite à 0°C pendant 15 minutes, introduit progressivement à 0°C, 1,9 g de 1R, cis 3-formyl 2,2-diméthyl cyclopropane carboxylate de terbutyle en solution dans 20 cm3 de chlorure de méthylène, agite pendant 30 heures à +20°C, filtre, concentre le filtrat à sec par distillation sous pression réduite, chromatographie le résidu sur silice en éluant par un mélange d'acétate d'éthyle et de chlorure de méthylène (1/1), puis par un mélange de chlorure de méthylène et d'hexane (3/7) et obtient 1,01 g de produit recherché.

Spectre IR (chloroforme) :

Absorption à 1 710cm-1 attribuée au 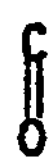 de l'ester ;

1 368cm-1 attribué au méthyle du terbutyle,
1 160cm-1 attribué à C-0-C.

Spectre de RMN (deutérochloroforme) :

Pics à 1,25 ppm attribué aux hydrogènes des méthyles géminés.
Pic à 1,47 ppm attribué aux hydrogènes du terbutyle.
Pics à 1,58 et à 1,85 ppm attribués aux hydrogènes en 1 et en 3 du cyclopropane.
Pics à 7,32-7,4 ppm attribués à l'hydrogène de l'éthyle.

Exemple 3 : 1R, trans 2,2-diméthyl 3-/2,2-diiodoéthényl/ cyclopropane carboxylate de (S) cyano (3-phénoxy phényl) méthyle.

Stade $A_1$ : Acide 1R, trans 2,2-diméthyl 3-/2,2-diiodo éthényl/ cyclopropane carboxylique.

On mélange 10,1 g de triphényl phosphine, 200 cm3 de chlorure de méthylène, introduit à 0°C 10,25 g de tétraiodométhane, agite pendant 15 minutes à 0°C, introduit progressivement 1,9 g de 3-formyl 2,2-diméthyl cyclopropane carboxylate de 1,1-diméthyléthyle en solution dans 20 cm3 de chlorure de méthylène, agite pendant 17 H à +20°C, ajoute 1,25 g de zinc électrolytique, agite pendant 10 minutes, filtre, concentre le filtrat à sec par distillation sous pression réduite, chromatographie le résidu sur silice en éluant par du chlorure de méthylène, puis par un mélange de chlorure de méthylène et d'acétate d'éthyle (1/1) et obtient 2,6 g de produit attendu, F = 100°C.

Spectre IR (chloroforme) :

Absorption à 3 505$^{cm-1}$ -OH, monomère et dimère ;

1 737$^{cm-1}$ 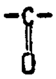 acide monomère ;

1 695$^{cm-1}$ 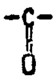 acide dimère.

Spectre de RMN (deuterochloroforme) :

Pics à 1,22-1,33 ppm attribués aux hydrogènes des méthyles géminés.
Pics à 1,64-1,73 ppm attribués à l'hydrogène en position 1 du cyclopropane.
Pics à 1,90-1,99 ppm et 2,02-2,12 ppm attribués à l'hydrogène en position 3 du cyclopropane.
Pics à 6,74-6,85 ppm attribués à l'hydrogène de l'éthényl.
Pic à 10,55 ppm attribué à l'hydrogène du carboxyle.

Stade $A_2$ : Acide 1R, trans 2,2-diméthyl 3-/2,2-diiodo éthényl/ cyclopropane carboxylique.

On mélange 2,2 g de 1 R, trans 2,2-diméthyl 3-/2,2-diiodoéthényl/ cyclopropane carboxylate de 1,1-diméthyl éthyle, 20 cm3 de toluène, porte au reflux, introduit 200 mg d'acide paratoluène sulfonique, maintient pendant 15 minutes au reflux, refroidit, concentre à sec par distillation sous pression réduite, chromatographie le résidu sur silice en éluant par un mélange d'hexane et d'acétate d'éthyle (6/4) et obtient 1,6 g de produit recherché, F = 101°C.

Stade B : 1R, trans 2,2-diméthyl 3-/2,2-diiodoéthényl/ cyclopropane carboxylate de (S) cyano (3-phénoxy phényl) méthyle.

On mélange 1,9 g d'acide 1 R, trans 2,2-diméthyl 3-/2,2-diiodo éthényl/ cyclopropane carboxylique, 1,3 g d'alcool (S) α- cyano 3-phénoxy benzylique, 20 mg de diméthyl amino-pyridine, 20 ml de chlorure de méthylène introduit à +5°C progressivement 1,45 g de dicyclohexylcarbodiimide en solution dans 10 cm3 de chlorure de méthylène, amène à +20°C, filtre, concentre le filtrat à sec par distillation sous pression réduite, chromatographie le résidu sur silice en éluant par un mélange d'hexane et d'acétate d'éthyle (9/1) et obtient 1,95 g de produit attendu.

Spectre IR (chloroforme) :

Absorption à 1 740$^{cm-1}$ attribué au 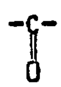 de l'ester

1 585$^{cm-1}$ et 1 485$^{cm-1}$ attribué à la fraction aromatique du type

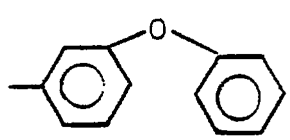

Spectre de RMN (deutérochloroforme) :

Pics à 1,2-1,25 ppm attribués aux hydrogènes des méthyles géminés.
Pics à 1,67-1,76 ppm attribués à l'hydrogène en position 1 du cyclopropane.
Pics de 1,93 à 2,15 ppm attribués à l'hydrogène en position 3 du cyclopropane.
Pic à 6,38 ppm attribué à l'hydrogène porté par le même carbone que le CN.
Pics à 6,75-6,87 ppm attribués à l'hydrogène de l'éthényle.
Pics de 6,92 à 7,58 ppm attribués aux hydrogènes des noyaux aromatiques.

<u>Exemple 4</u> : <u>1 R, trans 2,2-diméthyl 3-/2,2-diiodo éthényl/ cyclopropane carboxylate de (S) cyano 1-(3-phénoxy 4-fluorophényl) méthyle.</u>

On mélange 1,5 g d'acide 1 R, trans 2,2-diméthyl 3-/2,2-diiodio éthényl/ cyclopropane carboxylique, 855 mg d'alcool (S) cyano 3-phénoxy 4-fluoro phényl méthylique, 15 mg de 4-diméthylamino pyridine, 15 cm3 de chlorure de méthylène, introduit à +5°C une solution de 870 mg de dicyclohexyl carbodiimide dans 5 $cm^3$ de chlorure de méthylène, agite pendant 2 heures à +20°C, filtre, concentre le filtrat à sec par distillation sous pression réduite, chromatographie le résidu sur silice en éluant par un mélange de chlorure de méthylène et d'hexane (45/55) et obtient 1,4 g de produit attendu.

<u>Spectre IR (chloroforme)</u> :

Absorption à 1 740$^{cm-1}$ attribuée à 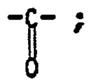 ;

1 610$^{cm-1}$
1 589$^{cm-1}$,
1 512$^{cm-1}$,
1 490$^{cm-1}$
attribués à la fraction aromatique du type

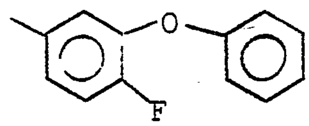

<u>Spectre de RMN (deuterochloroforme)</u> :

Pics à 1,19-1,22 ppm attribués aux hydrogènes des méthyles géminés.
Pics à 1,65-1,74 ppm attribués à l'hydrogène en position 1 du cyclopropane
Pics à 1,92-2,12 ppm attribués à l'hydrogène en position 3 du cyclopropane.
Pic à 6,33 ppmattribué à l'hydrogène porté par le même carbone que CN.
Pics à 6,73-6,85 ppm attribués à l'hydrogène de l'éthényle.
Pics de 6,88 à 7,5 ppm attribués aux hydrogènes des noyaux aromatiques.

<u>Exemple 5</u> :<u>1R,cis 2,2-diméthyl 3-/2,2-diiodo éthényl/ cyclopropane carboxylate de (S) cyano 1-(3-phénoxy 4-fluoro phényl) méthyle.</u>

<u>Stade A</u> : Acide 1R,cis 2,2-diméthyl 3-/2,2-diiodo éthényl/ cyclopropane carboxylique.

On mélange 930 mg de 1R, cis 2,2-diméthyl 3-/2,2-diiodo éthényl/cyclopropane carboxylate de terbutyle, 90 mg d'acide paratoluène sulfonique et 10 cm3 de toluène, porte le mélange réactionnel au reflux, maintient le reflux pendant 15 minutes, refroidit, concentre à sec par distillation sous pression réduite, chromatographie le résidu sur silice en éluant par un mélange d'acétate d'éthyle et d'hexane (3/7) et obtient 770 mg de produit recherché, F = 140°C.

<u>Spectre IR (chloroforme)</u> :

Absorption à 3 505$^{cm-1}$ OH, monomère et dimère ;

1 735$^{cm-1}$ 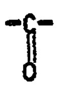 acide monomère ;

1 697$^{cm-1}$ 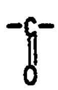 acide dimère.

Spectre de RMN (deutérochloroforme) :

Pic à 1,3 ppm attribué aux hydrogènes des méthyles géminés.
Pics à 1,63 et à 1,98 ppm attribués aux hydrogènes en position 1 et 3 du cyclopropane.
Pics de 7,13 à 7,42 ppm attribués à l'hydrogène de l'éthényle.

Stade B : 1R,cis 2,2-diméthyl 3-/2,2-diiodo éthényl/ cyclopropane carboxylate de (S) cyano 1-(3-phénoxy 4-fluoro phényl) méthyle.

On mélange 750 mg d'acide 1R, cis 2,2-diméthyl 3-/2,2-diiodo On mélange 750 mg d'acide 1R, cis 2,2-diméthyl 3-/2,2-diiodo éthényl/ cyclopropane carboxylique, 440 mg d'alcool (S) cyano 3-phénoxy 4-fluoro phényl) méthylique, 7,5 mg de 4-diméthylamino pyridine, 7,5 cm3 de chlorure de méthylène, introduit à +5°C progressivement une solution de 430 mg de dicyclohexyl carbodiimide dans 2,5 cm3 de chlorure de méthylène, agite pendant 2 heures à +20°C, filtre, concentre le filtrat à sec par distillation sous pression réduite, chromatographie sur silice en éluant par un mélange de chlorure de méthylène et d'hexane (1/1), lave par empâtage à l'hexane et obtient 700 mg de produit attendu F = 98°C.

Spectre IR (chloroforme) :

Absorption à 1 742$^{cm-1}$ attribué à 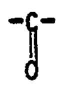 ;

1 610$^{cm-1}$,
1 590$^{cm-1}$,
1 510$^{cm-1}$,
1 490$^{cm-1}$,
attribués à la fraction aromatique du type

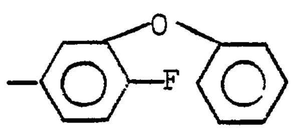

Spectre de RMN (deuterochloroforme) :

Pics à 1,17-1,25 ppm attribués aux hydrogènes des méthyles géminés.
Pics à 1,78 et à 1,98 ppm attribués aux hydrogènes en position 1 et 3 du cyclopropane.
Pic à 6,34 ppm attribué à l'hydrogène porté par le même carbone que CN.
Pics à 6,9-7,53 ppm attribués aux hydrogènes des noyaux aromatiques et à l'hydrogène de l'éthényle.

Exemple 6 : 1R, cis 2,2-diméthyl 3-/2,2-diiodoéthényl/ cyclopropane carboxylate de (S) cyano (3-phénoxy phényl) méthyle.

On opère comme indiqué au stade B de l'exemple 3, en utilisant au départ 1,9 g d'acide 1R,cis 2,2-diméthyl 3-/2,2-diiodoéthényl/ cyclopropane carboxylique (obtenu comme décrit à l'exemple 5). On obtient 1,95 g de produit brut que l'on chromatographie sur silice en éluant par un mélange de chlorure de méthylène et d'acétate d'éthyle (1/1), lave par empâtage dans l'acétonitrile, filtre, lave à l'éther et recristallise dans l'acétate d'éthyle. On obtient 1,7 g de produit recherché. F = 130°C.

Spectre IR (chloroforme) :

Absorption à 1 742 $^{cm-1}$ attribuée à 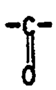 ;

1 587$^{cm-1}$ et 1 487$^{cm-1}$ fraction aromatique du type :

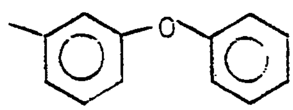

Spectre de RMN (deuterochloroforme) :

Pics à 1,22-1,29 ppm attribués aux hydrogènes des méthyles géminés.
Pics à 1,82-2,09 ppm attribués aux hydrogènes en position 1 et 3 du cyclopropane.
Pic à 6,43 ppm attribué à l'hydrogène porté par le même carbone que CN.
Pics de 6,98 à 7,6 ppm attribués aux hydrogènes des noyaux aromatiques et à l'hydrogène de l'éthényle.

Exemple 7 :1 R, trans 2,2-diméthyl 3/-(2,2-diiodoéthényl) cyclopropane carboxylate de /2-méthyl (1,1'-biphényl) 3-yl/méthyle.

On mélange 0,83 d'acide 1R, trans 2,2-diméthyl 3/2,2-diiodoéthényl cyclopropane carboxylique, 10 cm3 de chlorure de méthylène, 10 mg de diméthylamino pyridine, 0,43 g d'alcool /2-méthyl-(1,1'-biphényl)-3-yl/méthylique, ajoute en une seule fois à 20¤C 0,447 g de dicyclohexyl carbodiimide, élimine par filtration l'urée formée, concentre à sec par distillation sous pression réduite, chromatographie le résidu sur silice dans un mélange d'hexane et d'acétate d'éthyle (9/1), chromatographie le résidu sur silice en éluant par un mélange d'hexane et d'acétate d'éthyle (9/1) et obtient 0,95 g de produit recherché.

Spectre IR (chloroforme) :

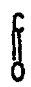 ester à 1 720$^{cm-1}$

aromatique à 1 590$^{cm-1}$
aromatique à 1 498$^{cm-1}$

Spectre de RMN ($CHCl_3$) :

hydrogènes des méthyles géminés : 1,22-1,32 ppm
hydrogène en position 1 du cylopropyle : 1,71-1,8 ppm
hydrogène en position 3 du cylopropyle : 1,99 à 2,13 ppm
hydrogène du méthyle en 2 : 2,25 ppm
hydrogène de

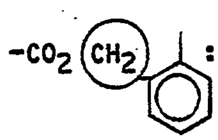

5,23 ppm
hydrogène éthylénique : 6,77-6,88 ppm
hydrogènes aromatiques : 7,22 à 7,56 ppm.

Exemple 8 : 1R, cis 2,2-diméthyl 3-(2,2-diiodo éthényl) cyclopropane carboxylate de /2-méthyl -(1,1'-biphényl) 3-yl/ méthyle.

On mélange 1 g d'acide 1R, cis 2,2 diméthyl 3/2,2-diiodo éthényl) cyclopropane carboxylique, 20 cm3 de chlorure de méthylène, 10 mg de diméthylamino pyridine, 0,55 g d'alcool de 2-méthyl-(1,1'-biphényl)-3-yl/ méthylique, ajoute en une seule fois 0,58 g de dicyclohexycarbodiimide, agite pendant 2 heures à 20¤C, élimine par filtration l'urée formée, lave la phase organique à l'eau, concentre à sec par distillation sous pression réduite, chromatographie le résidu sur silice en éluant par un mélange hexane-acétate d'éthyle (9/1) et obtient 1,3 g de produit recherché.

Spectre IR ($CHCl_3$) :

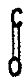 ester à 1 720$^{cm-1}$

aromatique à 1 585$^{cm-1}$
aromatique à 1 495$^{cm-1}$

Spectre de RMN ($CDCl_3$) :

hydrogènes des méthyles géminés : 1,26-1,28 ppm
hydrogène en position 1 et en 3 du cyclopropyle : 1,55 à 2,02 ppm
hydrogène du méthyle en 2 : 2,31 ppm
hydrogène de

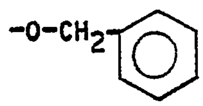 :

5,17 ppm.
Les hydrogènes aromatiques et le proton éthylénique : 7,15 à 7,48 ppm.

Analyse: 572, 288 $C_{22}H_{22}O_2I_2$

| | C % | H % | I % |
|---|---|---|---|
| Calculés | 46,18 | 3,87 | 44,35 |
| Trouvés | 47,2 | 4,0 | 42,4 |

Exemple 9 : 1Scis 2,2-diméthyl 3-/2,2-diiodo éthényl/ cyclopropane carboxylate de 1,1-diméthyl éthyle.

On introduit à 0°C 10 g de tétraiodométhane dans une solution renfermant 10,1 g de triphénylphosphine et 180 ml de chlorure de méthylène. On maintient, sous agitation, pendant 20 minutes à 0°C, et introduit 1,9 g de 1Scis 2,2-diméthyl 3-formyl cyclopropane carboxylate de 1,1-diméthyl éthyle, et 20 cm3 de chlorure de méthylène. On maintient ensuite sous agitation pendant 17 heures. On lave, sèche et évapore à sec. On obtient un résidu que l'on reprend dans 250 cm3 d'éther isopropylique. On filtre, évapore le filtrat et obtient 3,85 g d'un produit que l'on chromatographie sur silice en éluant par le mélange chlorure de méthylène hexane (3-7). On obtient ainsi 2,3 g de produit recherché fondant à 54°C.

Exemple 10 : 1Scis 2,2-diméthyl 3-/2,2-diiodo éthényl/ cyclopropane carboxylate de R cyano 1-(3-phénoxy, 4-fluorophényl) méthyle.

Stade A : Acide 1Scis 2,2-diméthyl 3-/2,2-diiodo éthényl/ cyclopropane carboxylique.

On chauffe au reflux une solution renfermant 2,15 g de produit préparé au stade A, 20 cm3 de toluène et 200 mg d'acide paratoluène sulfonique. On refroidit et dilue avec 30 cm3 d'acétate d'éthyle. On lave les phases organiques et les sèche. On évapore à sec sous pression réduite. On obtient 1,85 g de produit que l'on chromatographie sur silice en éluant par le mélange hexane acétate d'éthyle 7-3. On obtient ainsi 1,55 g de produit recherché fondant à 139°C.

Stade B : 1Scis 2,2-diméthyl 3-/2,2-diiodo éthényl/ cyclopropane carboxylate de R cyano 1-(3-phénoxy 4-fluorophényl) méthyle.

On introduit à 0°C 460 mg de dicyclohexylcarbodiimide dans 5 cm3 de chlorure de méthylène dans une solution renfermant 0,8 g d'acide 1Scis 2,2-diméthyl 3-/2,2-diiodoéthényl/cyclopropane carboxylique, 458 mg d'alcool (R) cyano 3-phénoxy 4-fluoro phényl/ méthylique, 9 mg de diméthyl aminopyridine, et 8 cm3 de chlorure de méthylène. On filtre et évapore le solvant sous pression réduite. On obtient un produit que l'on chromatographie sur silice en éluant par le mélange chlorure de méthylène-hexane ( 1 -1). On obtient une résine que l'on chromatographi e à nouveau et obtient 780 mg du produit recherché fondant à 95°C. $/\alpha/_D = -25° \pm 1$ (c = 0,9% $CHCl_3$)

Spectre IR Absorption à 1742 $cm^{-1}$ attribué à 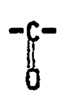

1590 $cm^{-1}$
1513 $cm^{-1}$ attribué à la fraction aromatique
1491 $cm^{-1}$

Spectre RMN

H des méthyles géminés : 1,2 - 1,26 ppm
H en position 1 et en 3 du cyclopropyle : 1,66 à 2 ppm
H éthylénique : 6,95 à 7,57 ppm
H porté par le même carbone que CN : 6,38 ppm

Exemple 11 :1R, cis 2,2-diméthyl 3(2,2-diiodo éthényl) cyclopropane carboxylate de S cyano 1-(3-phénoxy 4-fluorophényl) méthyle et la 1S,cis 2,2-diméthyl 3-(2,2-diiodo éthényl) cyclopropane carboxylate de R cyano 1-(3-phénoxy 4-fluorophényl méthyle en mélange 50 - 50.

En opérant comme précédemment à partir du mélange de l'acide 1R,cis 2,2-diméthyl 3/2,2-diiodo éthényl) cyclopropane carboxylique et de son isomère 1S,cis et de l'alcool RS cyano 1-(3-phénoxy 4-fluorophényl) méthylique, on obtient après séparation par chromatographie le racémique (1Rcis S - 1Scis R) fondant à 104°C.

Exemple 12 : 1R,cis 2,2-diéthyl 3-(2,2-diiodoéthényl) cyclopropane carboxylate de (S) cyano 1-(3-phénoxy phényl) méthyle et le 1S,cis 2,2-diméthyl 3-(2,2-diiodoéthényl) cyclopropane carboxylate de R cyano 1-(3-phénoxy phényl) méthyle en mélange 50 - 50.

En opérant comme à l'exemple 11, à partir de l'alcool RS cyano 1-(3-phénoxy phényl) méthylique, on obtient le produit fondant à 116°C.

Exemples 13 à 22

En opérant selon le procédé de l'invention, on a préparé les produits suivants :

- le 1R, trans 2,2-diméthyl 3-/2,2-diiodo éthényl/ cyclopropane carboxylate de pentafluoro phényl méthyle.
/ α /$_D$ = +4°5 ±0°5 c = 1,4 % $CHCl_3$
- le 1R,trans 2,2-diméthyl 3-/2,2-diiodo éthényl/ cyclopropane carboxylate de 1-(3-phénoxy phényl) méthyle.
/ α /$_D$ = +8°5 ±0°5 c = 1,5 % $CHCl_3$
- le 1R,trans 2,2-diméthyl 3-(2,2-diiodo éthényl) cyclopropane carboxylate de (S) 3-(2-propényl) 2-méthyl 4-oxo 2-cyclopentényle.
/ α /$_D$ = -2° +1 c = 1% $CHCl_3$
- le 1R,trans 2,2-diméthyl 3-(2,2-diiodo éthényl) cyclopropane carboxylate de méthyl 1-(6-phénoxy 2-pyridyl) méthyle.
/ α /$_D$ = +25°5 ±1° c = 1% $CHCl_3$
- le 1R,trans 2,2-diméthyl 3-(2,2-diiodo éthényl) cyclopropane carboxylate d' cyano 1-(6-phénoxy 2-pyridyl) méthyle.
/ α /$_D$ = +16° ±0°5 c = 1,5% $CHCl_3$
- le 1R,cis 2,2-diméthyl 3-(2,2-diiodo éthényl) cyclopropane carboxylate de (S) 3-(2-propényl) 2-méthyl 4-oxo 2-cyclopentényle
/ α /$_D$ = +13° +1°5 c = 0,6 % $CHCl_3$
- le 1R,cis 2,2-diméthyl 3 (2,2-diiodo éthényl) cyclopropane carboxylate de cyano 1-(6-phénoxy 2-pyridyl) méthyle.
/ α /$_D$ = -5° ±1° c = 0,9 % $CHCl_3$
- le 1R,cis 2,2-diméthyl 3-(2,2-diiodo éthényl) cyclopropane carboxylate de méthyl 1-(6-phénoxy 2-pyridyl) méthyle.
/ α /$_D$ = +7° ±1° c = 1% $CHCl_3$
- le 1R,cis 2,2-diméthyl 3-(2,2-diiodo éthényl) cyclopropane carboxylate de 1-(3-phénoxy phényl) méthyle.
/ α /$_D$ = -11° ±1° c = 1% $CHCl_3$
- le 1R,cis 2,2-diméthyl 3-(2,2-diiodo éthényl) cyclopropane carboxylate de pentafluoro phényl méthyle.
/ α /$_D$ = -14° ±0,°5 c = 2% $CHCl_3$

Exemple 23 : Préparation d'un concentré soluble.

On effectue un mélange homogène de :

| | |
|---|---|
| Produit de l'exemple 5 | 0,25 g |
| Butoxyde de pipéronyle | 1 g |
| Tween 80 | 0,25 g |
| Topanol A | 0,1 g |
| Eau | 98,4 g |

Exemple 24 : Préparation d'un concentré émulsifiable

On mélange intimement:

| | |
|---|---|
| Produit de l'exemple 5 | 0,015 g |
| Butoxyde de pipéronyle | 0,5 g |
| Topanol A | 0,1 g |
| Xylène | 95,885 g |
| Tween 80 | 3,5 g |

Exemple 25 : Préparation d'un concentré émulsifiable

On mélange de façon homogène :

| | |
|---|---|
| Produit de l'exemple 5 | 1,5 g |
| Tween 80 | 20 g |
| Topanol A | 0,2 g |
| Xylène | 78,4 g |

Exemple 26 : Préparation d'une composition fumigène

On mélange de façon homogène :

| | |
|---|---|
| Produit de l'exemple 5 | 0,25 g |
| Poudre de tabu | 25 g |
| Poudre de fueilles de cèdre | 40 g |
| Poudre de bois de pin | 33,75 g |
| Vert brillant | 0,5 g |
| p. nitrophénol | 0,5 g |

Exemple 27 : Préparation d'un concentré soluble.

On effectue un mélange homogène de :

| | |
|---|---|
| Produit de l'exemple11 | 0,25 g |
| Butoxyde de pipéronyle | 1 g |
| Tween 80 | 0,25 g |
| Topanol A | 0,1 g |
| Eau | 98,4 g |

Exemple 28 : Préparation d'un concentré émulsifiable.

On mélange intimement :

| | |
|---|---|
| Produit de l'exemple 7 | 0,015 g |
| Butoxyde de pipéronyle | 0,5 g |
| Topanol A | 0,1 g |
| Xylène | 95,885 g |
| Tween 80 | 3,5 g |

Exemple 29 : Préparation d'un concentré émulsifiable

On mélange de façon homogène :

| | |
|---|---|
| Produit de l'exemple 8 | 1,5 g |
| Tween 80 | 20 g |
| Topanol A | 0,2 g |
| Xylène | 78,4 g |

ETUDE BIOLOGIQUE

ETUDE DE L'ACTIVITE PESTICIDE DES COMPOSES SELON L'INVENTION

1°/. : Etude de l'effet léthal sur mouches domestiques.

Les insectes tests sont des mouches domestiques femelles âgées de 4/5 jours. On opère par application topique de 1µ l de solution acétonique du produit sur le thorax dorsal des insectes à l'aide du micro manipulateur d'Arnold. On utilise 50 individus par dose et par traitement. On effectue le contrôle de mortalité vingt-quatre heures après traitement.
Le résultat obtenu exprimé en DL50 ou dose (en nanogrammes) nécessaire pour tuer 50% des insectes, est le suivant :

| Produit de l'exemple | $DL_{50}$ en ng par individu |
|---|---|
| 4 | 3,8 |
| 5 | 1,6 |
| 6 | 2,0 |
| 11 | 1,92 |

Conclusion : Dans le test utilisé, les produits des exemples 4, 5, 6 et 11 présentent une activité remarquable.

2°/. :Etude de l'activité par contact tarsal sur blatte germanique.

Les insectes testés sont des mâles de blatte germanique (Blatella germanica). On opère par dépôt d'une solution acétonique de concentration déterminée sur le fond d'une boite de Petri de 20 cm de diamètre. Après séchage, on laisse séjourner 20 mâles de blattes par concentration durant 1 heure, puis on transfère les insectes sur milieu sain et on contrôle leur mortalité à 24 h, 48 h, 3 et 5 jours.

Le résultat est exprimé en concentration léthale 50 (CL 50).

| Produit de l'exemple | $CL_{50}$ en $mg/m^2$ |
|---|---|
| 3 | 0,15 |
| 4 | 0,152 |
| 5 | 0,025 |
| 6 | 0,062 |
| 11 | 0,121 |

<u>Conclusion</u> : Dans le test utilisé les produits des exemples 3, 4, 5, 6 et 11 présentent une activité remarquable.

3¤/. : <u>Etude de l'activité de choc sur mouche domestique</u>.

Les insectes tests sont des mouches domestiques femelles âgées de 4/5 jours. On opère par pulvérisation directe en chambre de Kearns et March en utilisant comme solvant un mélange en volumes égaux d'acétone et d'isopar L (quantité de solution utilisée 2 × 0,2 $cm^3$). On utilise environ 50 insectes par dose de traitement. On effectue les contrôles toutes les minutes jusqu'à 10 minutes, puis à 15 minutes et l'on détermine le KT 50 par les méthodes habituelles.

Les résultats obtenus sont les suivants :

| Produit de l'exemple | $KT_{50}$ en minutes – concentration 1 g/l |
|---|---|
| 3 | 6,9 |
| 4 | 5,3 |
| 5 | 6,1 |
| 6 | 6,4 |
| 11 | 6,9 |

<u>Conclusion</u> : Dans le test utilisé, les produits des exemples 3, 4, 5, 6 et 11 présentent une bonne activité.

4¤/. : <u>Etude de l'effet léthal sur larves de Spodoptera littoralis</u>.

Les essais sont effectués par application topique d'une solution acétonique du produit à tester à l'aide du micro manipulateur d'Arnold sur le thorax dorsal des larves. On utilise 15 larves par dose de produit à tester. Les larves utilisées sont des larves du quatrième stade larvaire, c'est-à-dire âgées d'environ 10 jours lorsqu'elles sont élevées à 24¤C et 65% d'humidité relative. Après traitement, les individus sont placés sur un milieu nutritif artificiel (milieu de Poitout).

On effectue le contrôle des mortalités 48 heures après traitement.

| Produit de l'exemple | $DL_{50}$ en ng par individu |
|---|---|
| 5 | 1,16 |
| 11 | 4,8 |

<u>Conclusion</u> : le produit des exemples 5 et 11 dans le test utilisé, sont doués d'une remarquable activité.

5¤/. <u>Etude de l'effet léthal sur Acanthoscelides obtectus.</u>

Les essais sont effectués par application topique, de manière analogue à celle utilisée pour les larves de spodoptera littoralis.

Les résultats obtenus sont les suivants :

| Produit de l'exemple | $DL_{50}$ en ng par individu |
|---|---|
| 4 | 11,7 |
| 5 | 6,1 |
| 6 | 11,3 |
| 11 | 10,7 |

Conclusion : Dans le test utilisé, les produits des exemples 4, 5, 6 et 11 présentent une activité remarquable.

6⌧ Etude de l'effet létal sur Aphis cracivora

En opérant comme au paragraphe 5, sur Aphis cracivora, on a obtenu le résultat suivant exprimé en concentration létal 50 ($CL_{50}$).

| Produit de l'exemple | $CL_{50}$ |
|---|---|
| 11 | 0,46 mg/l |

Conclusion : Dans le test utilisé, le produit de l'exemple 11 présente une bonne activité.

**Revendications pour les Etats contractants BE, CH, DE, FR, GB, IT, LU, NL**

1/. Les composés de formule générale (I) sous toutes leurs formes stéréoisomères ou sous forme de mélanges de stéréoisomères :

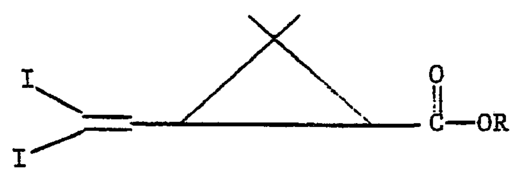

(I)

dans laquelle R représente le reste d'un alcool utilisé dans le domaine des pyréthrinoïdes.

2/. Les composés de formule générale (I), selon la revendication 1, sous toutes leurs formes stéréoisomères ou sous forme de mélanges de stéréoisomères, caractérisés en ce que R représente :
- soit un radical alcoyle, linéaire ou ramifié, contenant de 1 à 8 atomes de carbone,
- soit un radical benzyle,
- soit un groupement de formule :

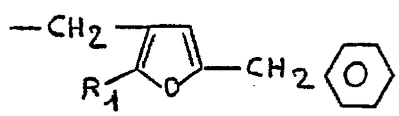

dans laquelle $R_1$ représente un atome d'hydrogène ou un radical méthyle,
- soit un groupement de formule :

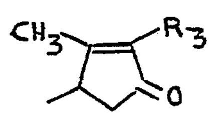

dans laquelle $R_3$ représente un radical aliphatique organique contenant de 2 à 6 atomes de carbone et une ou plusieurs insaturations carbone-carbone et plus particulièrement les radicaux $-CH_2 = CH = CH_2$, $-CH_2-CH = CH-CH_3$, $-CH_2-CH = CH-C_2H_5$, $-CH_2-CH = CH -CH = CH_2$, $CH_2-C = CH$,
- soit un groupement de formule :

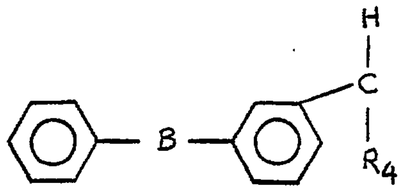

dans laquelle B représente un groupement, C = O ou un atome d'oxygène et $R_4$ représente un atome d'hydrogène, un radical méthyle, un groupement -C≡N, un groupement -C≡CH, un groupement 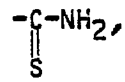

- soit un groupement de formule :

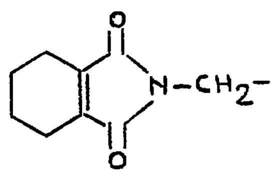

- soit un groupement de formule :

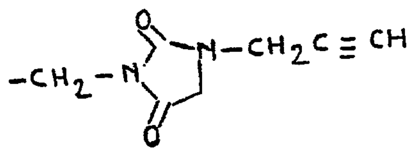

- soit un groupement de formule :

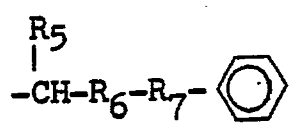

dans laquelle $R_5$ représente un atome d'hydrogène ou un radical -CN,

$R_6$ représente un radical thiazolyle dans lequel la liaison 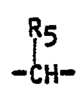 peut se trouver à l'une quelconque des positions disponibles, $R_7$ représente un radical $-CH_2-$ ou un atome d'oxygène :
- soit un groupement de formule :

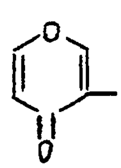

- soit un groupement de formule :

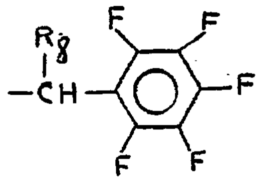

dans laquelle $R_8$ représente un atome d'hydrogène ou un radical CN,
- soit un groupement de formule :

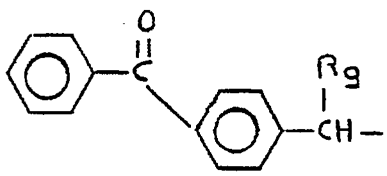

dans laquelle $R_9$ représente un atome d'hydrogène ou un radical -CN
- soit un groupement de formule :

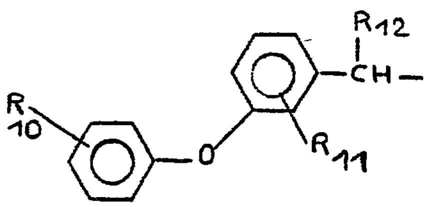

dans laquelle $R_{12}$ représente un atome d'hydrogène, un radical méthyle, éthynyle ou cyano et $R_{10}$ et $R_{11}$ différents l'un de l'autre représentent un atome d'hydrogène, de fluor, de chlore ou de brome,
- soit un groupement de formule

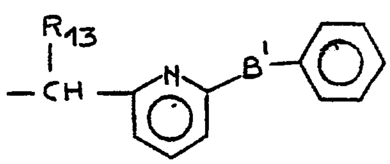

dans laquelle $R_{13}$ représente un atome d'hydrogène, un radical méthyle, éthynyle ou cyano et B′ représente un atome d'oxygène ou de soufre,
- soit un groupement de formule :

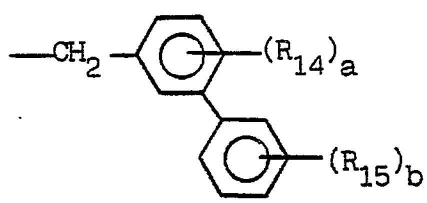

dans laquelle $R_{14}$ représente un atome d'halogène, un radical alcoyle renfermant de 1 à 6 atomes de carbone ou un radical trifluorométhyle, $R_{15}$ représente un atome d'halogène, un radical alcoyle renfermant de 1 à 6 atomes de carbone de carbone, un radical alcoxy renfermant de 1 à 6 atomes de carbone ou un radical trifluorométhyle, a est 0, 1, 2, 3 ou 4 et b est 0, 1,2, 3, 4 ou 5.

3/. Les composés de formule générale (I) selon les revendications 1 ou 2 sous toutes leurs formes stéréoisomères ou sous forme de mélange de stéréoisomères, caractérisés en ce que R représente un groupement de formule :

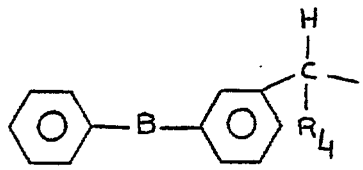

dans laquelle B représente un groupement C = O ou de l'oxygène et $R_4$ représente de l'hydrogène, un radical méthyle, un groupement C ≡ N, un groupement -C ≡ CH, ou un groupement 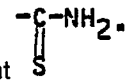

4/. Les composés de formule générale (I), selon les revendications 1 ou 2, sous toutes leurs formes stéréoisomères ou sous forme de mélanges de stéréoisomères, caractérisés en ce que R représente un groupement de formule :

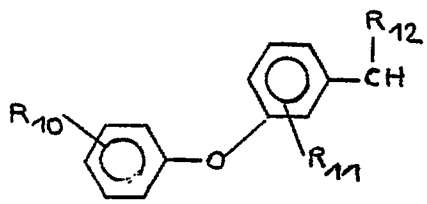

dans laquelle $R_{12}$ représente un atome d'hydrogène, un radical méthyle, éthynyle ou cyano et $R_{10}$ et $R_{11}$ différents l'un de l'autre représentent de l'hydrogène ou un atome de fluor, de chlore ou de brome.

5/. Les composés de formule générale (I) selon les revendications 1 ou 2 sous toutes leurs formes stéréoisomères ou sous forme de mélanges de stéréoisomères, caractérisés en ce que R représente un groupement de formule :

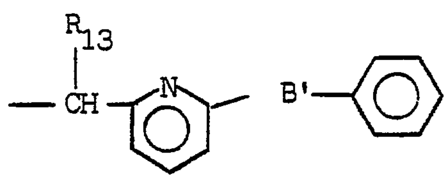

dans laquelle $R_{13}$ représente un atome d'hydrogène, un radical méthyle, éthynyle ou cyano et B' représente un atome d'oxygène ou de soufre.

6/. Les composés de formule générale (I) selon les revendications 1 ou 2 sous toutes leurs formes stéréoisomères ou sous forme de mélanges de stereoisomères, caractérisés en ce que R représente un groupement de formule :

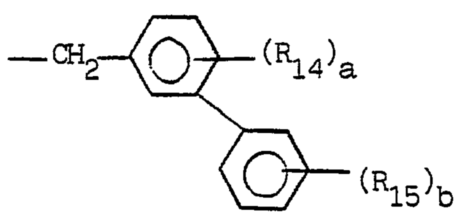

dans laquelle $R_{14}$ représente un atome d'halogène, un radical alcoyle renfermant de 1 à 6 atomes de carbone ou un radical trifluorométhyle, $R_{15}$ représente un atome d'halogène, un radical alcoyle renfermant de 1 à 6 atomes de carbone, un radical alcoxy renfermant de 1 à 6 atomes de carbone ou un radical trifluorométhyle, a est 0, 1, 2, 3 ou 4 et b est 0, 1, 2, 3 4 ou 5.

7/. Les composés de formule générale (I) sous toutes leurs formes stéréoisomères ou sous forme de mélanges de stéréoisomères, selon l'une quelconque des revendications 1 à 6, caractérisées en ce que R représente un groupement de formule :

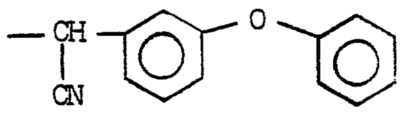
,
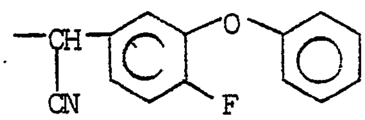

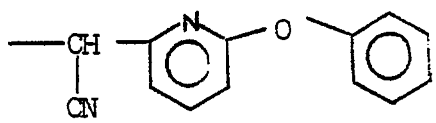
ou
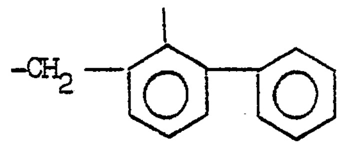

8/. Les composés dont les noms suivents :
- le 1R,cis 2,2-diméthyl 3-/2,2-diiodo éthényl/ cyclopropane carboxylate de (S) cyano (3-phénoxy 4-fluoro phényl) méthyle,
- le 1R,cis 2,2-diméthyl 3-(2,2-diiodoéthényl) cyclopropane carboxylate de (S) cyano 1-(3-phénoxy 4-fluorophényl) méthyle et le 1S,cis 2,2-diméthyl 3-(2,2-diiodoéthényl) cyclopropane carboxylate de (R) cyano 1-(3-phénoxy 4-fluorophényl) méthyle en mélange 50 - 50.

9/. Procédé de préparation des composés de formule générale (I), telle que définie à la revendication 1 sous toutes leurs formes stéréoisomères ou sous forme de mélanges de stéréoisomères, caractérisé en ce que l'on fait réagir le tétraiiodométhane très pur, à une température d/environ 0°C et la triphénylphosphine, au sein d'un solvant organique, avec un composé de formule (II) :

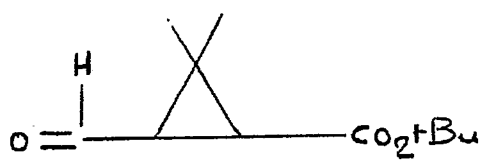
(II)

sous toutes ses formes stéréoisomères ou sous forme de mélanges de stéréoisomères, soumet le composé résultant de formule (III) :

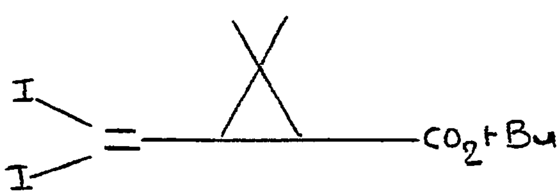
(III)

à l'action de l'acide paratoluène sulfonique au sein d'un solvant organique pour obtenir un composé de formule (IV) :

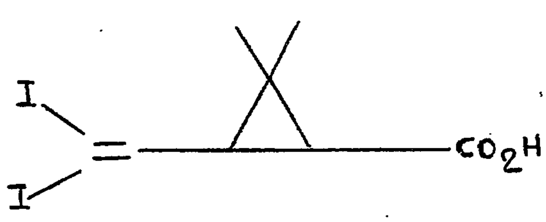
(IV)

que l'on transforme éventuellement en dérivé fonctionnel tel que chlorure d'acide, anhydride ou anhydride mixte, fait réagir l'acide de formule (IV) ou son dérivé fonctionnel sur l'alcool de formule:
R-OH
ou sur un dérivé fonctionnel de cet alcool pour obtenir le produit de formule générale (I) correspondant :

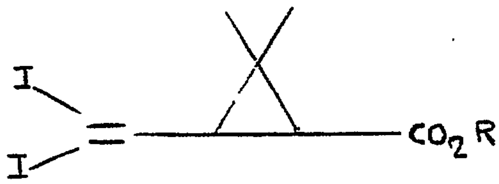

(I)

10/. Procédé de préparation selon la revendication 9 caractérisé en ce que
- le solvant organique au sein duquel on fait réagir le tétraiodométhane et la triphényl-phosphine avec un composé de formule (II) est un solvant aliphatique chloré tel que le chlorure de méthylène,
- le solvant organique au sein duquel ont fait réagir l'acide paratoluène sulfonique sur un composé de formule (III) est un solvant aromatique tel que le benzène, le toluène ou le xylène, le dérivé fonctionnel de l'acide de formule (IV) est le chlorure de l'acide et l'on fait réagir le chlorure d'acide sur l'alcool R-OH en présence d'une base tertiaire,
- on fait réagir l'acide de formule (IV) avec l'alcool ROH, en présence de dicyclohexyl carbodiimide ou de diisopropyl carbodiimide et de diméthyl amino pyridine.

11/. Procédé de préparation selon la revendication 9, caractérisé en ce que l'on fait réagir le composé de formule (II) :

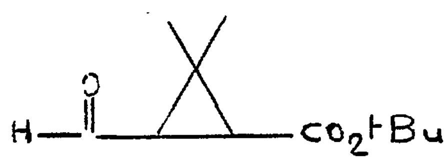

(II)

avec le tétraiodométhane, la triphénylphosphine et le zinc au sein d'un solvant organique pour obtenir un composé de formule (IV) :

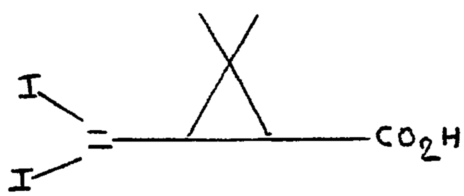

(IV)

puis termine la synthèse comme à la revendication 9.

12/. Procédé de préparation selon la revendication 11, caractérisé en ce que le solvant organique utilisé est un solvant aliphatique chloré tel que le chlorure de méthylène.

13/.Les composés de formule I tels que définis à l'une quelconque des revendications 1 à 8, pour leur utilisation à la lutte contre les parasites des végétaux, les parasites des locaux et les parasites des animaux à sang chaud.

14/. Les compositions pesticides destinées à la lutte contre les parasites des végétaux, les parasites des locaux et les parasites des animaux à sang chaud, caractérisées en ce qu'elles renferment comme principe actif au moins un composé défini à l'une quelconque des revendications 1 à 8.

15/. Les compositions insecticides renfermant comme principe actif au moins l'un des composés définis à l'une quelconque des revendications 1 à 8.

16/. Les compositions acaricides destinées à lutter contre les acariens parasites des végétaux, renfermant comme principe actif au moins l'un des composés de formule générale (I), définis à l'une quelconque des revendications 1 à 8.

17/. Les compositions nématicides renfermant comme principe actif, au moins l'un des composés définis à l'une quelconque des revendications 1 à 8.

18/. Les compositions acaricides destinées à lutter contre les acariens parasites des animaux, renfermant comme principe actif au moins l'un des composés définis à l'une quelconque des revendications 1 à 8.

19/. Les compositions destinées à l'alimentation animale, caractérisées en ce qu'elles renferment comme principe actif au moins l'un des composés définis à l'une quelconque des revendications 1 à 8.

20/. Associations douées d'activité insecticide, acaricide ou nématicide, caractérisées en ce qu'elles contiennent comme matière active, d'une part, un au moins des composés de formule générale (I), et

d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides chrysanthémiques, par les esters d'alcools 5-benzyl 3-furyl méthylique des acides 2,2-diméthyl 3-(2-oxo 3-tétrahydrothiophénylidène méthyl) cyclopropane-1-carboxyliques, par les esters d'alcools 3-phénoxy benzyliques et d'alcools α-cyano 3-phénoxy benzyliques des acides 2,2-diméthyl 3-(2,2-dichlorovinyl) cyclopropane-1-carboxyliques, par les esters d'alcools α-cyano 3-phénoxy benzyliques d'acides 2,2-diméthyl 3-(2,2-dibromovinyl) cyclopropane-1-carboxyliques, par les esters d'alcools 3-phénoxy benzylique des acides 2-parachlorophényl 2-isopropyl acétiques, par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides 2,2-diméthyl 3-(1,2,2,2-tétrahaloéthyl) cyclopropane-1-carboxyliques, dans lesquels "halo" représente un atome de fluor, de chlore ou de brome, étant entendu que les composés I peuvent exister sous toutes leurs formes stéréoisomères possibles de même que les copules acides et alcools des esters pyréthrinoïdes ci-dessus.

**Revendications pour l'Etat contractant ES**

1.- Procédé pour préparer les composés de formule générale (I) sous toutes leurs formes stéréoisomères ou sous forme de mélanges de stéréoisomères :

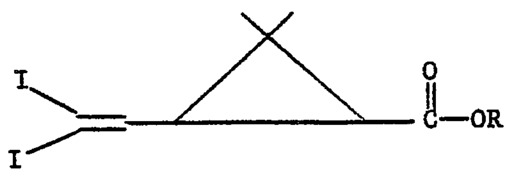

(I)

dans laquelle R représente le reste d'un alcool utilisé dans le domaine des pyréthrinoïdes, caractérisé en ce que l'on fait réagir le tétraiiodométhane très pur, à une température d'environ 0°C et la la triphénylphosphine, au sein d'un solvant organique, avec un composé de formule (II) :

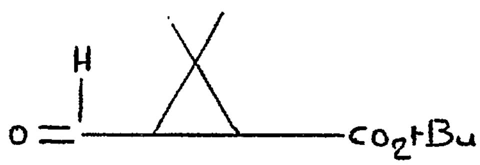

(II)

sous toutes ses formes stéréoisomères ou sous forme de mélanges de stéréoisomères, soumet le composé résultant de formule (III) :

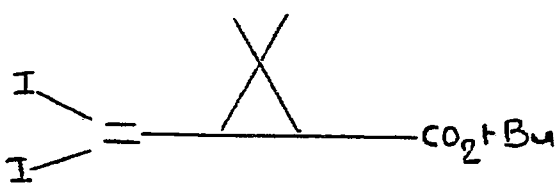

(III)

à l'action de l'acide paratoluène sulfonique au sein d'un solvant organique pour obtenir un composé de formule (IV) :

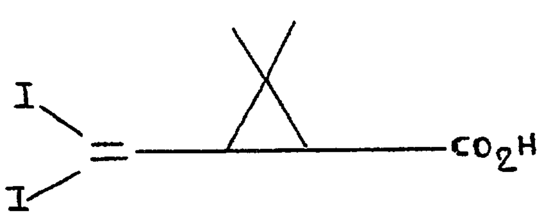

(IV)

que l'on transforme éventuellement en dérivé fonctionnel tel que chlorure d'acide, anhydride ou anhydride mixte, fait réagir l'acide de formule (IV) ou son dérivé fonctionnel sur l'alcool de formule :
R-OH
ou sur un dérivé fonctionnel de cet alcool pour obtenir le produit de formule générale (I) correspondant:

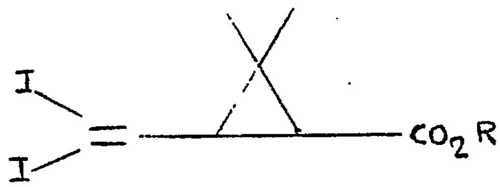
(I)

2.- Procédé de préparation selon la revendication 1 caractérisé en ce que
- le solvant organique au sein duquel on fait réagir le tetraiodométhane et la triphényl-phosphine avec un composé de formule (II) est un solvant aliphatique chloré tel que le chlorure de méthylène,
- le solvant organique au sein duquel ont fait réagir l'acide paratoluène sulfonique sur un composé de formule (III) est un solvant aromatique tel que le benzène, le toluène ou le xylène,
-le dérivé fonctionnel de l'acide de formule (IV) est le chlorure de l'acide et l'on fait réagir le chlorure d'acide sur l'alcool R-OH en présence d'une base tertiaire,
- on fait réagir l'acide de formule (IV) avec l'alcool ROH, en présence de dicyclohexyl carbodiimide ou de diisopropyl carbodiimide et de diméthyl amino pyridine.

3.- Procédé de préparation selon la revendication 9, caractérisé en ce que l'on fait réagir le composé de formule (II) :

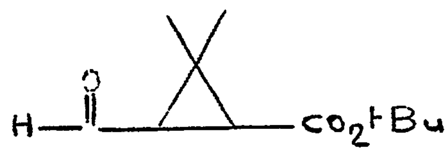
(II)

avec le tétraiodométhane, la triphénylphosphine et le zinc au sein d'un solvant organique pour obtenir un composé de formule (IV) :

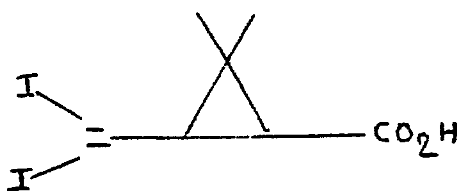
(IV)

puis termine la synthèse comme à la revendication 1.

4.- Procédé de préparation selon la revendication 3, caractérisé en ce que le solvant organique utilisé est un solvant aliphatique chloré tel que le chlorure de méthylène.

5.- Procédé selon l'une quelconque des revendications 1 à 4, caractérisé en ce que l'on utilise au départ un alcool de formule R-OH ou un dérivé fonctionnel de cet alcool, formule dans laquelle R représente :
- soit un radical alcoyle, linéaire ou ramifié, contenant de 1 à 8 atomes de carbone,
- soit un radical benzyle,
- soit un groupement de formule :

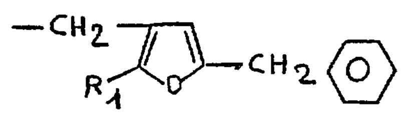

dans laquelle $R_1$ représente un atome d'hydrogène ou un radical méthyle,
- soit un groupement de formule :

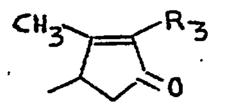

dans laquelle $R_3$ représente un radical aliphatique organique contenant de 2 à 6 atomes de carbone et une ou plusieurs insaturations carbone-carbone et plus particulièrement les radicaux $-CH_2-CH=CH_2$, $-CH_2-CH=CH-CH_3$, $-CH_2-CH=CH-C_2H_5$, $-CH_2-CH=CH-CH=CH_2$, $CH_2-C\equiv CH$,
- soit un groupement de formule :

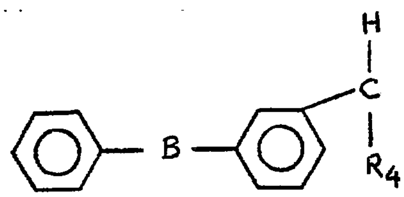

dans laquelle B représente un groupement, C = O ou un atome d'oxygène et $R_4$ représente un atome d'hydrogène, un radical méthyle, un groupement -C≡N, un groupement -C≡CH, un groupement 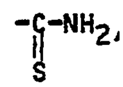
- soit un groupement de formule :

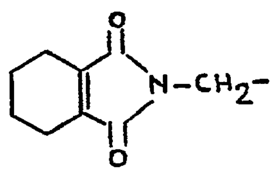

- soit un groupement de formule :

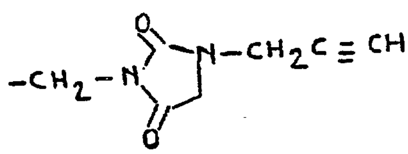

- soit un groupement de formule :

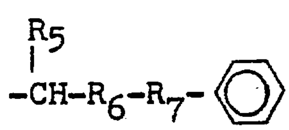

dans laquelle $R_5$ représente un atome d'hydrogène ou un radical -CN,

$R_6$ représente un radical thiazolyle dans lequel la liaison 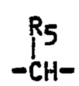 peut se trouver à l'une quelconque des positions disponibles, $R_7$ représente un radical $-CH_2-$ ou un atome d'oxygène :
- soit un groupement de formule :

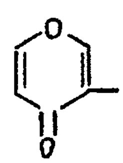

- soit un groupement de formule :

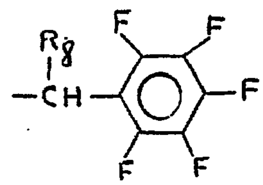

dans laquelle $R_8$ représente un atome d'hydrogène ou un radical CN,
- soit un groupement de formule :

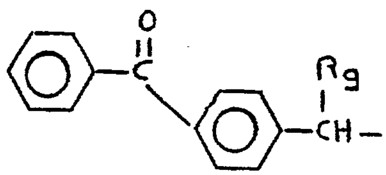

dans laquelle $R_9$ représente un atome d'hydrogène ou un radical -CN
- soit un groupement de formule :

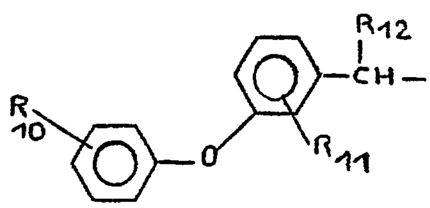

dans laquelle $R_{12}$ représente un atome d'hydrogène, un radical méthyle, éthynyle ou cyano et $R_{10}$ et $R_{11}$ différents l'un de l'autre représentent un atome d'hydrogène, de fluor, de chlore ou de brome,
- soit un groupement de formule :

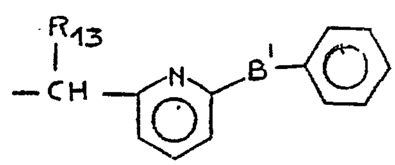

dans laquelle $R_{13}$ représente un atome d'hydrogène, un radical méthyle, éthynyle ou cyano et B′ représente un atome d'oxygène ou de soufre,
- soit un groupement de formule :

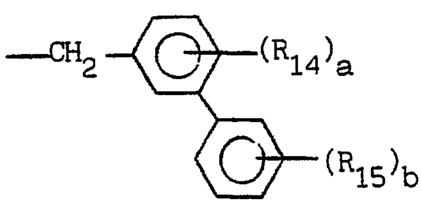

dans laquelle $R_{14}$ représente un atome d'halogène, un radical alcoyle renfermant de 1 à 6 atomes de carbone ou un radical trifluorométhyle, $R_{15}$ représente un atome d'halogène, un radical alcoyle renfermant de 1 à 6 atomes de carbone de carbone, un radical alcoxy renfermant de 1 à 6 atomes de carbone ou un radical trifluorométhyle, a est 0, 1, 2, 3 ou 4 et b est 0, 1,2, 3, 4 ou 5.

6.- Procédé selon l'une quelconque des revendications 1 à 4, caractérisé en ce que l'on utilise au départ un alcool de formule R-OH ou un dérivé fonctionnel de cet alcool, formule dans laquelle R représente un groupement de formule :

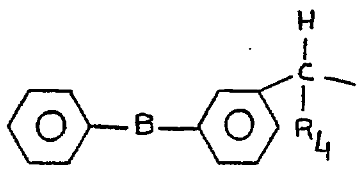

dans laquelle B représente un groupement C = O ou de l'oxygène et $R_4$ représente de l'hydrogène, un radical méthyle, un groupement C ≡ N, un groupement -C ≡ CH, ou un groupement 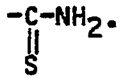

7.- Procédé selon l'une quelconque des revendications 1 à 4, caractérisé en ce que l'on utilise au départ un alcool de formule R-OH ou un dérivé fonctionnel de cet alcool, formule dans laquelle R représente un groupement de formule :

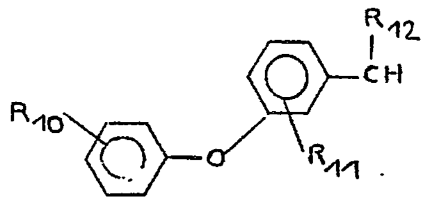

dans laquelle $R_{12}$ représente un atome d'hydrogène, un radical méthyle, éthynyle ou cyano et $R_{10}$ et $R_{11}$ différents l'un de l'autre représentent de l'hydrogène ou un atome de fluor, de chlore ou de brome.

8.- Procédé selon l'une quelconque des revendications 1 à 4, caractérisé en ce que l'on utilise au départ un alcool de formule R-OH ou un dérivé fonctionnel de cet alcool, formule dans laquelle R représente un groupement de formule :

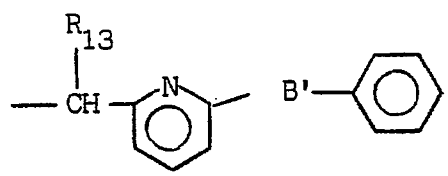

dans laquelle $R_{13}$ représente un atome d'hydrogène, un radical méthyle, éthynyle ou cyano et B′ représente un atome d'oxygène ou de soufre.

9.- Procédé selon l'une quelconque des revendications 1 à 4, caractérisé en ce que l'on utilise au départ un alcool de formule R-OH ou un dérivé fonctionnel de cet alcool, formule dans laquelle R représente un groupement de formule :

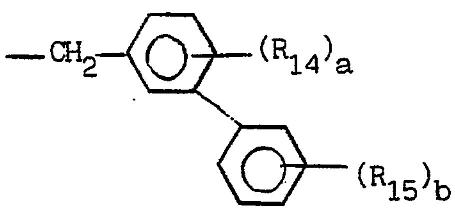

dans laquelle $R_{14}$ représente un atome d'halogène, un radical alcoyle renfermant de 1 à 6 atomes de carbone ou un radical trifluorométhyle, $R_{15}$ représente un atome d'halogène, un radical alcoyle renfermant de 1 à 6 atomes de carbone, un radical alcoxy renfermant de 1 à 6 atomes de carbone ou un radical trifluorométhyle, a est 0, 1, 2, 3 ou 4 et b est 0, 1, 2, 3 4 ou 5.

10.- Procédé selon l'une quelconque des revendications 1 à 4, caractérisé en ce que l'on utilise au départ un alcool de formule R-OH ou un dérivé fonctionnel de cet alcool, formule dans laquelle R représente un groupement de formule :

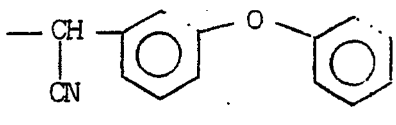 , 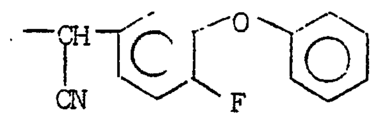

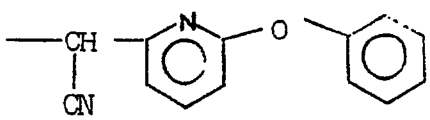 ou 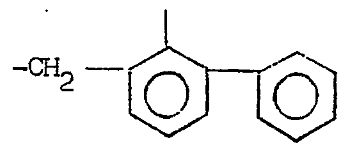

11.- Procédé selon l'une quelconque des revendications 1 à 4, caractérisé en ce que l'on utilise au départ un dérivé de formule (II) dans laquelle la configuration est 1R,cis ou 1RS,cis et un alcool de formule R-OH, ou un dérivé fonctionnel de cet alcool, formule dans laquelle R représente un groupement de formule :

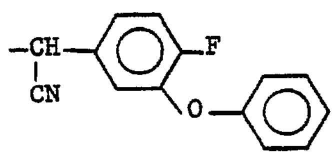

de configuration (S) ou (R,S).

**Revendications pour l'Etat contractant AT**

1.- Procédé pour préparer les composés de formule générale (I) sous toutes leurs formes stéréoisomères ou sous forme de mélanges de stéréoisomères :

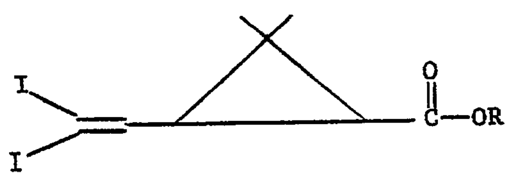

(I)

dans laquelle R représente le reste d'un alcool utilisé dans le domaine des pyréthrinoïdes, caractérisé en ce que l'on fait réagir le tétraiiodométhane très pur, à une température d'environ 0°C et la triphénylphosphine, au sein d'un solvant organique, avec un composé de formule (II) :

(II)

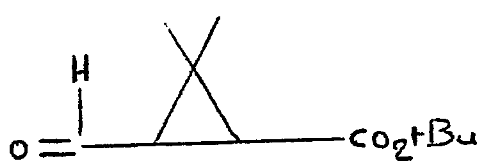

sous toutes ses formes stéréoisomères ou sous forme de mélanges de stéréoisomères, soumet le composé résultant de formule (III) :

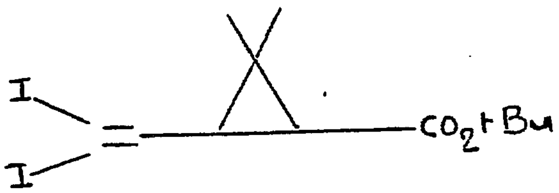

(III)

à l'action de l'acide paratoluène sulfonique au sein d'un solvant organique pour obtenir un composé de formule (IV) :

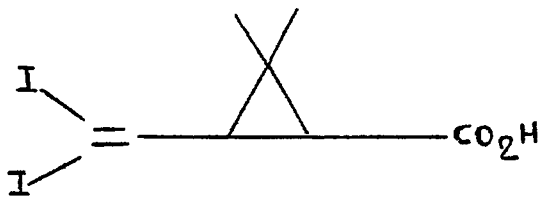

(IV)

que l'on transforme éventuellement en dérivé fonctionnel tel que chlorure d'acide, anhydride ou anhydride mixte, fait réagir l'acide de formule (IV) ou son dérivé fonctionnel sur l'alcool de formule :
R-OH
ou sur un dérivé fonctionnel de cet alcool pour obtenir le produit de formule générale (I) correspondant :

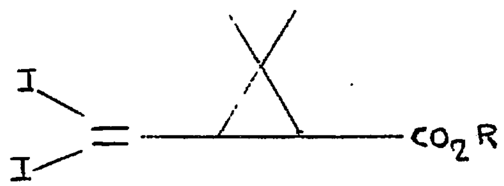

(I)

2.- Procédé de préparation selon la revendication 1 caractérisé en ce que
- le solvant organique au sein duquel on fait réagir le tétraiodométhane et la triphényl-phosphine avec un composé de formule (II) est un solvant aliphatique chloré tel que le chlorure de méthylène,
- le solvant organique au sein duquel ont fait réagir l'acide paratoluène sulfonique sur un composé de formule (III) est un solvant aromatique tel que le benzène, le toluène ou le xylène, le dérivé fonctionnel de l'acidé de formule (IV) est le chlorure de l'acide et l'on fait réagir le chlorure d'acide sur l'alcool R-OH en présence d'une base tertiaire,
- on fait réagir l'acide de formule (IV) avec l'alcool ROH, en présence de dicyclohexyl carbodiimide ou de diisopropyl carbodiimide et de diméthyl amino pyridine.

3.- Procédé de préparation selon la revendication 9, caractérisé en ce que l'on fait réagir le composé de formule (II) :

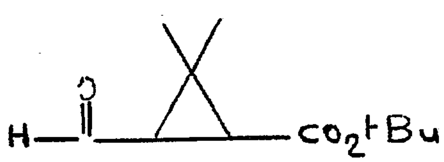

(II)

avec le tétraiodométhane, la triphénylphosphine et le zinc au sein d'un solvant organique pour obtenir un composé de formule (IV) :

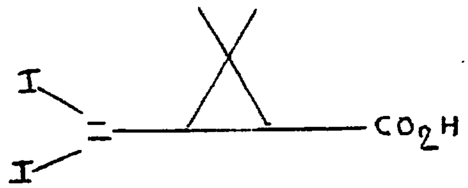

(IV)

puis termine la synthèse comme à la revendication 1.

4.- Procédé de préparation selon la revendication 3, caractérisé en ce que le solvant organique utilisé est un solvant aliphatique chloré tel que le chlorure de méthylène.

5.- Procédé selon l'une quelconque des revendications 1 à 4, caractérisé en ce que l'on utilise au départ un alcool de formule R-OH ou un dérivé fonctionnel de cet alcool, formule dans laquelle R représente :

- soit un radical alcoyle, linéaire ou ramifié, contenant de 1 à 8 atomes de carbone,
- soit un radical benzyle,
- soit un groupement de formule :

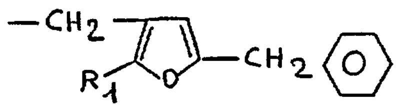

dans laquelle $R_1$ représente un atome d'hydrogène ou un radical méthyle,

- soit un groupement de formule :

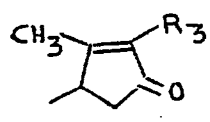

dans laquelle $R_3$ représente un radical aliphatique organique contenant de 2 à 6 atomes de carbone et une ou plusieurs insaturations carbone-carbone et plus particulièrement les radicaux $-CH_2 = CH = CH_2$, $-CH_2-CH = CH-CH_3$, $-CH_2-CH = CH-C_2H_5$, $-CH_2-CH = CH -CH = CH_2$, $CH_2-C \equiv CH$,

- soit un groupement de formule :

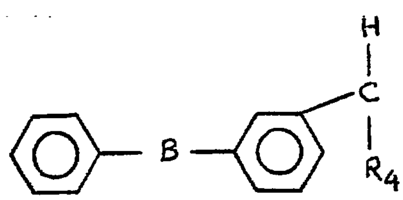

dans laquelle B représente un groupement, C = O ou un atomê d'oxygène et $R_4$ représente un atome d'hydrogène, un radical méthyle, un groupement -C≡N, un groupement -C≡CH, un groupement 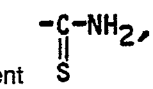

- soit un groupement de formule :

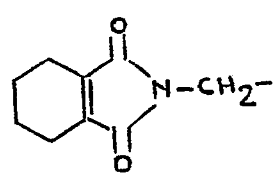

- soit un groupement de formule :

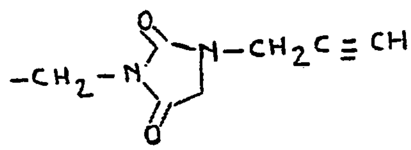

- soit un groupement de formule :

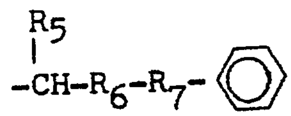

dans laquelle $R_5$ représente un atome d'hydrogène ou un radical -CN,

$R_6$ représente un radical thiazolyle dans lequel la liaison 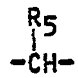 peut se trouver à l'une quelconque des positions disponibles, $R_7$ représente un radical $-CH_2-$ ou un atome d'oxygène :

- soit un groupement de formule :

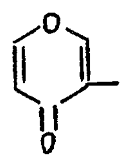

- soit un groupement de formule :

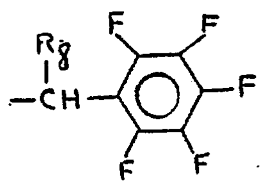

dans laquelle $R_8$ représente un atome d'hydrogène ou un radical CN,

- soit un groupement de formule :

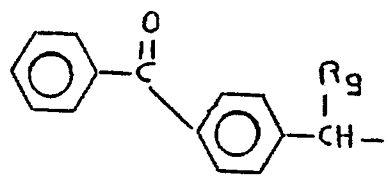

dans laquelle $R_9$ représente un atome d'hydrogène ou un radical -CN

- soit un groupement de formule :

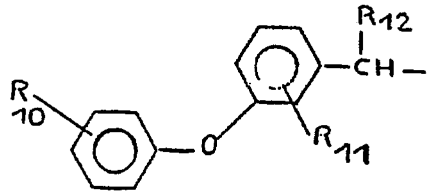

dans laquelle $R_{12}$ représente un atome d'hydrogène, un radical méthyle, éthynyle ou cyano et $R_{10}$ et $R_{11}$ différents l'un de l'autre représentent un atome d'hydrogène, de fluor, de chlore ou de brome,

- soit un groupement de formule :

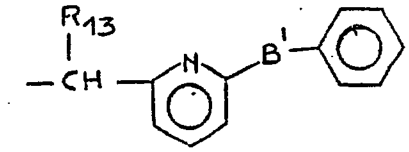

dans laquelle $R_{13}$ représente un atome d'hydrogène, un radical méthyle, éthynyle ou cyano et B' représente un atome d'oxygène ou de soufre,
- soit un groupement de formule :

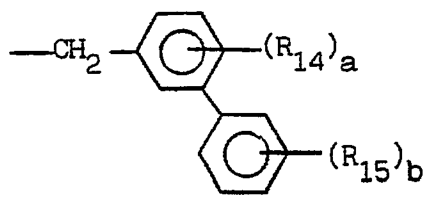

dans laquelle $R_{14}$ représente un atome d'halogène, un radical alcoyle renfermant de 1 à 6 atomes de carbone ou un radical trifluorométhyle, $R_{15}$ représente un atome d'halogène, un radical alcoyle renfermant de 1 à 6 atomes de carbone de carbone, un radical alcoxy renfermant de 1 à 6 atomes de carbone ou un radical trifluorométhyle, a est 0, 1, 2, 3 ou 4 et b est 0, 1,2, 3, 4 ou 5.

6.- Procédé selon l'une quelconque des revendications 1 à 4, caractérisé en ce que l'on utilise au départ un alcool de formule R-OH ou un dérivé fonctionnel de cet alcool, formule dans la quelle R représente un groupement de formule :

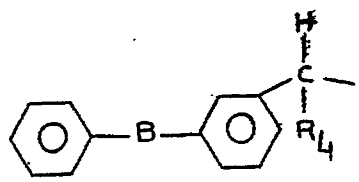

dans laquelle B représente un groupement C = O ou de l'oxygène et $R_4$ représente de l'hydrogène, un radical méthyle, un groupement C ≡ N, un groupement -C ≡ CH, ou un groupement 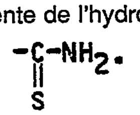

7.- Procédé selon l'une quelconque des revendications 1 à 4, caractérisé en ce que l'on utilise au départ un alcool de formule R-OH ou un dérivé fonctionnel de cet alcool, formule dans laquelle R représente un groupement de formule :

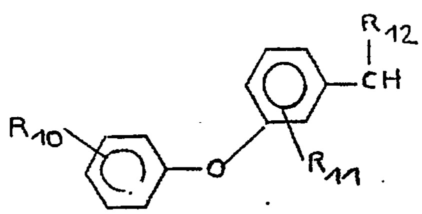

dans laquelle $R_{12}$ représente un atome d'hydrogène, un radical méthyle, éthynyle ou cyano et $R_{10}$ et $R_{11}$ différents l'un de l'autre représentent de l'hydrogène ou un atome de fluor, de chlore ou de brome.

8.- Procédé selon l'une quelconque des revendications 1 à 4, caractérisé en ce que l'on utilise au départ un alcool de formule R-OH ou un dérivé fonctionnel de cet alcool, formule dans laquelle R représente un groupement de formule :

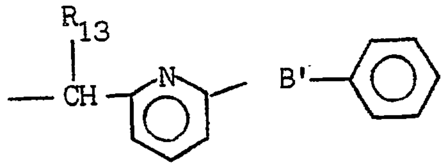

dans laquelle $R_{13}$ représente un atome d'hydrogène, un radical méthyle, éthynyle ou cyano et B' représente un atome d'oxygène ou de soufre.

9.- Procédé selon l'une quelconque des revendications 1 à 4, caractérisé en ce que l'on utilise au départ un alcool de formule R-OH ou un dérivé fonctionnel de cet alcool, formule dans laquelle R représénte un groupement de formule :

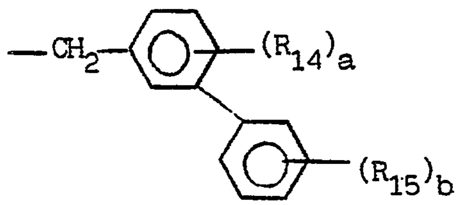

dans laquelle $R_{14}$ représente un atome d'halogène, un radical alcoyle renfermant de 1 à 6 atomes de carbone ou un radical trifluorométhyle, $R_{15}$ représente un atome d'halogène, un radical alcoyle renfermant de 1 à 6 atomes de carbone, un radical alcoxy renfermant de 1 à 6 atomes de carbone ou un radical trifluorométhyle, a est 0, 1, 2, 3 ou 4 et b est 0, 1, 2, 3 4 ou 5.

10.- Procédé selon l'une quelconque des revendications 1 à 4, caractérisé en ce que l'on utilise au départ un alcool de formule R-OH ou un dérivé fonctionnel de cet alcool, formule dans laquelle R représente un groupement de formule :

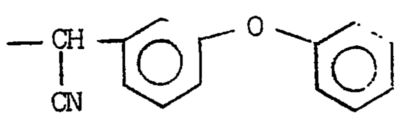 , 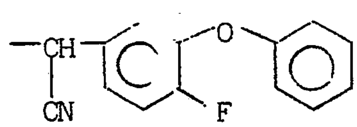

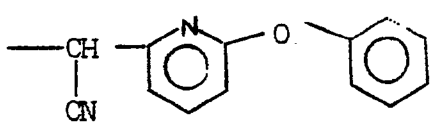 ou 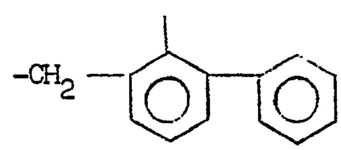

11.- Procédé selon l'une quelconque des revendications 1 à 4, caractérisé en ce que l'on utilise au départ un dérivé de formule (II) dans laquelle la configuration est 1R,cis ou 1RS,cis et un alcool de formule R-OH, ou un dérivé fonctionnel de cet alcool, formule dans laquelle R représente un groupement de formule :

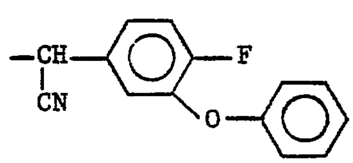

de configuration (S) ou (R,S).

12.- Les compositions pesticides destinées à la lutte contre les parasites des végétaux, les parasites des locaux et les parasites des animaux à sang chaud, caractérisées en ce qu'elles renferment comme principe actif au moins un composé défini à l'une quelconque des revendications 1 à 11.

13.- Les compositions insecticides renfermant comme principe actif, au moins l'un des composés définis à l'une quelconque des revendications 1 à 11.

14.- Les compositions acaricides destinées à lutter contre les acariens parasites des végétaux, renfermant comme principe actif au moins l'un des composés de formule générale (I), définis à l'une quelconque des revendications 1 à 11.

15.- Les compositions nématicides renfermant comme principe actif, au moins l'un des composés définis à l'une quelconque des revendications 1 à 11.

16/. Les compositions acaricides destinées à lutter contre les acariens parasites des animaux, renfermant comme principe actif au moins l'un des composés définis à l'une quelconque des revendications 1 à 11.

17.- Les compositions destinées à l'alimentation animale, caractérisées en ce qu'elles renferment comme principe actif au moins l'un des composés définis à l'une quelconque des revendications 1 à 11.

18.- Compositions douées d'activité insecticide, acaricide ou nématicide, caractérisées en ce qu'elles contiennent comme matière active, d'une part, un au moins des composés de formule générale (I), et d'autre part, un au moins des esters pyréthrinoïdes choisis dans le groupe constitué par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides chrysanthémiques, par les esters d'alcools 5-benzyl 3-furyl méthylique des acides 2,2-diméthyl 3-(2-oxo 3-tétrahydrothiophénylidène méthyl) cyclopropane-1-carboxyliques, par les esters d'alcools 3-phénoxy benzyliques et d'alcools α-cyano 3-phénoxy benzyliques des acides 2,2-diméthyl 3-(2,2-dichlorovinyl) cyclopropane-1-carboxyliques, par les esters d'alcools α-cyano 3-phénoxy benzyliques d'acides 2,2-diméthyl 3-(2,2-dibromovinyl) cyclopropane-1-carboxyliques, par les esters d'alcools 3-phénoxy benzylique des acides 2-parachlorophényl 2-isopropyl acétiques, par les esters d'alléthrolones, d'alcool 3,4,5,6-tétrahydrophtalimido méthylique, d'alcool 5-benzyl 3-furyl méthylique, d'alcool 3-phénoxy benzylique et d'alcools α-cyano 3-phénoxy benzyliques des acides 2,2-diméthyl 3-(1,2,2,2-tétrahaloéthyl) cyclopropane-1-carboxyliques, dans lesquels "halo" représente un atome de fluor, de chlore ou de brome, étant entendu que les composés I peuvent exister sous toutes leurs formes stéréoisomères possibles de même que les copules acides et alcools des esters pyréthrinoïdes ci-dessus.

**Patentansprüche für die Vertragsstaaten BE, CH, DE, FR, GB, IT, LU, NL**

1. Verbindungen der allgemeinen Formel (I) in sämtlichen ihrer stereoisomeren Formen oder in Form von Gemischen der Stereoisomeren

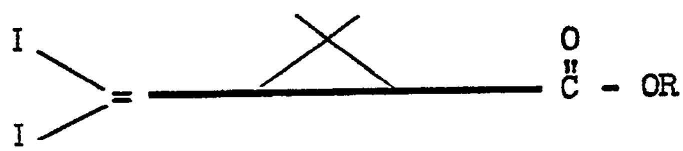 (I)

worin R den Rest eines auf dem Gebiet der Pyrethrinoide verwendeten Alkohols bedeutet.

2. Verbindungen der allgemeinen Formel (I) gemäß Anspruch 1 in sämtlichen ihrer stereoisomeren Formen oder in Form von Gemischen der Stereoisomeren, dadurch gekennzeichnet, daß R bedeutet:
entweder einen linearen oder verzweigten Alkylrest mit 1 bis 8 Kohlenstoffatomen
oder einen Benzylrest
oder eine Gruppe der Formel

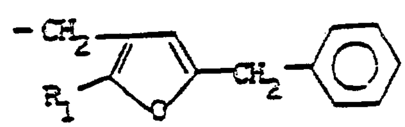

worin $R_1$ für ein Wasserstoffatom oder eine Methylgruppe steht,
oder eine Gruppe der Formel

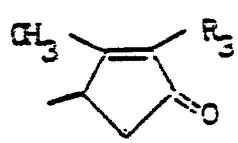

worin $R_3$ einen organischen, aliphatischen Rest mit 2 bis 6 Kohlenstoffatomen und einer oder mehreren Kohlenstoff-Kohlenstoff-Unsättigungen und insbesondere die Reste:
$-CH_2=CH=CH_2$,
$-CH_2-CH=CH-CH_3$,
$-CH_2-CH=CH-C_2H_5$,

$-CH_2-CH=CH-CH=CH_2$,
$-CH_2-C=CH$, wiedergibt,
oder eine Gruppe der Formel

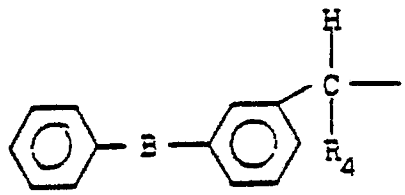

worin B eine Gruppe C=O oder ein Sauerstoffatom bedeutet und $R_4$ ein Wasserstoffatom, eine Methylgruppe, eine Gruppe $-C{\equiv}N$, eine Gruppe $-C{\equiv}CH$, eine Gruppe 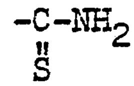 darstellt,
oder eine Gruppe der Formel

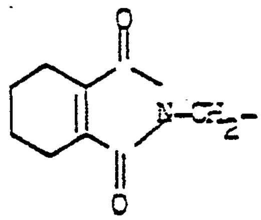

oder eine Gruppe der Formel

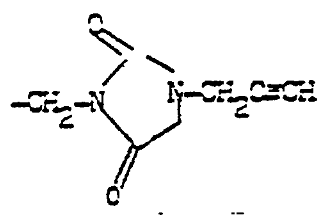

oder eine Gruppe der Formel

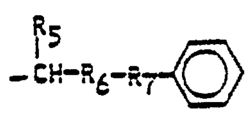

worin $R_5$ ein Wasserstoffatom oder einen Rest $-CN$ bedeutet, $R_6$ für eine Thiazolylgruppe steht, in der die Bindung 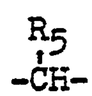 sich an irgendeiner der verfügbaren Positionen befinden kann, $R_7$ einen Rest $-CH_2-$ oder ein Sauerstoffatom darstellt,
oder eine Gruppe der Formel

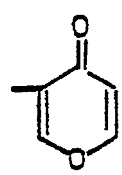

oder eine Gruppe der Formel

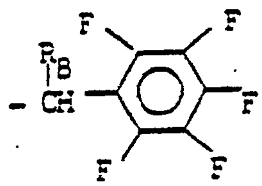

worin $R_8$ für ein Wasserstoffatom oder einen Rest CN steht oder eine Gruppe der Formel

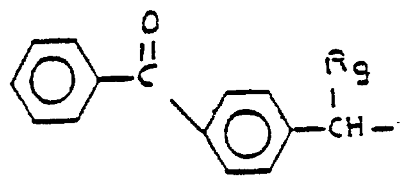

worin $R_9$ für ein Wasserstoffatom oder einen Rest –CN steht, oder eine Gruppe der Formel

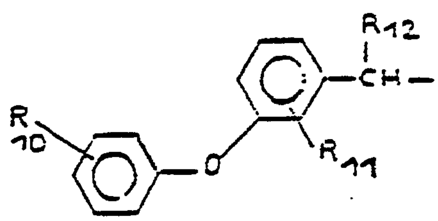

worin $R_{12}$ für ein Wasserstoffatom, eine Methyl-, Ethinyl- oder Cyanogruppe steht und $R_{10}$ und $R_{11}$, die voneinander verschieden sind, ein Wasserstoff-, Fluor-, Chlor- oder Bromatom bedeuten, oder eine Gruppe der Formel

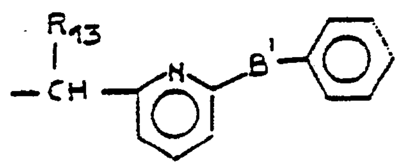

worin $R_{13}$ für ein Wasserstoffatom, eine Methyl-, Ethinyl- oder Cyanogruppe steht und B′ ein Sauerstoff- oder Schwefelatom wiedergibt, oder eine Gruppe der Formel

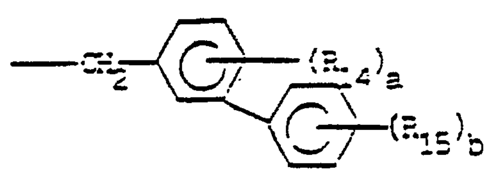

worin $R_{14}$ ein Halogenatom, eine Alkylgruppe mit 1 bis 6 Kohlenstoffatomen oder eine Trifluormethylgruppe bedeutet, $R_{15}$ für ein Halogenatom, eine Alkylgruppe mit 1 bis 6 Kohlenstoffatomen, eine Alkoxygruppe mit 1 bis 6 Kohlenstoffatomen oder eine Trifluormethylgruppe steht, a für 0, 1, 2, 3 oder 4 steht und b 0, 1, 2, 3, 4 oder 5 ist.

3. Verbindungen der allgemeinen Formel (I) gemäß Anspruch 1 oder 2 in sämtlichen ihrer stereoisomeren Formen oder in Form von Gemischen der Stereoisomeren, dadurch gekennzeichnet, daß R eine Gruppe der Formel

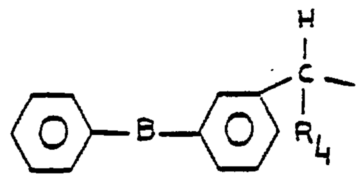

wiedergibt, worin B eine Gruppe C=O oder Sauerstoff bedeutet und $R_4$ für Wasserstoff, eine Methylgruppe, eine Gruppe C≡N, eine Gruppe –C≡CH oder eine Gruppe 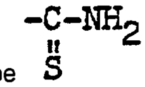 steht.

4. Verbindungen der allgemeinen Formel (I) gemäß den Ansprüchen 1 oder 2 in sämtlichen ihrer stereoisomeren Formen oder in Form von Gemischen der Stereoisomeren, dadurch gekennzeichnet, daß R eine Gruppe der Formel

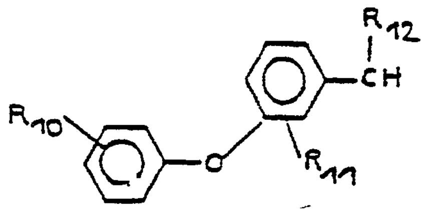

wiedergibt, worin $R_{12}$ ein Wasserstoffatom, eine Methyl-, Ethinyl- oder Cyanogruppe bedeutet und $R_{10}$ und $R_{11}$, die voneinander verschieden sind, für Wasserstoff oder ein Fluor-, Chlor- oder Bromatom stehen.

5. Verbindungen der allgemeinen Formel (I) gemäß Anspruch 1 oder 2 in sämtlichen ihrer stereoisomeren Formen oder in Form von Gemischen der Stereoisomeren, dadurch gekennzeichnet, daß R eine Gruppe der Formel

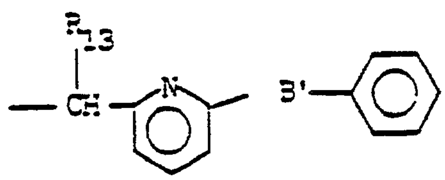

wiedergibt, worin $R_{13}$ für ein Wasserstoffatom, eine Methyl-, Ethinyl- oder Cyanogruppe steht und B′ ein Sauerstoff- oder Schwefelatom darstellt.

6. Verbindungen der allgemeinen Formel (I) gemäß den Ansprüchen 1 oder 2 in sämtlichen ihrer stereoisomeren Formen oder in Form von Gemischen der Stereoisomeren, dadurch gekennzeichnet, daß R eine Gruppe der Formel

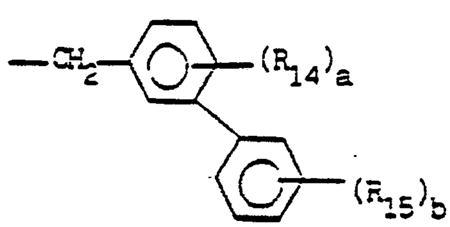

wiedergibt, worin $R_{14}$ ein Halogenatom, einen Alkylrest mit 1 bis 6 Kohlenstoffatomen oder eine Trifluormethylgruppe bedeutet, $R_{15}$ für ein Halogenatom, eine Alkylgruppe mit 1 bis 6 Kohlenstoffatomen, eine Alkoxygruppe mit 1 bis 6 Kohlenstoffatomen oder eine Trifluormethylgruppe steht, a 0, 1, 2, 3 oder 4 ist und b für 0, 1, 2, 3, 4 oder 5 steht.

7. Verbindungen der allgemeinen Formel (I) in sämtlichen ihrer stereoisomeren Formen oder in Form von Gemischen der Stereoisomeren gemäß einem der Ansprüche 1 bis 6, dadurch gekennzeichnet, daß R eine Gruppe der Formel

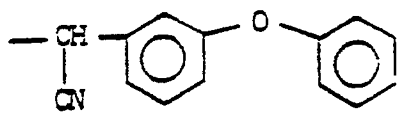 , 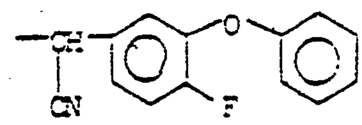

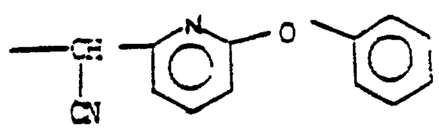 oder 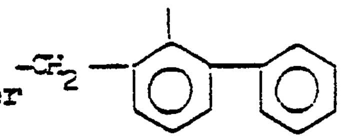

wiedergibt.

8. Verbindungen mit den folgenden Bezeichnungen:
(S)-Cyano-(3-phenoxy-4-fluorphenyl)-methyl-1R,cis-2,2-dimethyl-3-(2,2-dijodethenyl)-cyclopropan-carboxylat sowie
(S)-Cyano-1-(3-phenoxy-4-fluorphenyl)-methyl-1R,cis-2,2-dimethyl-3-)2,2-dijodethenyl)-cyclopropan-carboxylat und
(R)-Cyano-1-(3-phenoxy-4-fluorphenyl)-1S,cis-2,2-dimethyl-3-(2,2-dijodethenyl)-cyclopropan-carboxylat in einem 50/50-Gemisch.

9. Verfahren zur Herstellung der Verbindungen der allgemeinen Formel (I) gemäß Anspruch 1 in sämtlichen ihrer stereoisomeren Formen oder in Form von Gemischen der Stereoisomeren, dadurch gekennzeichnet, daß man sehr reines Tetrajodmethan bei einer Temperatur von etwa 0°C und Triphenylphosphin in einem organischen Lösungsmittel mit einer Verbindung der Formel (II)

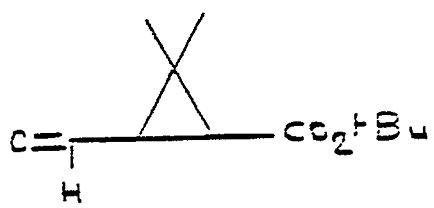

(II)

in sämtlichen ihrer stereoisomeren Formen oder in Form von Gemischen der Stereoisomeren umsetzt, die resultierende Verbindung der Formel (III)

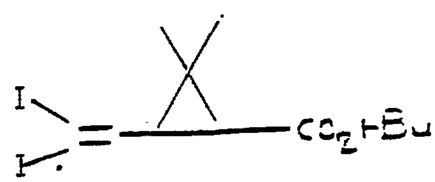

(III)

der Einwirkung von p-Toluolsulfonsäure in einem organischen Lösungsmittel unterzieht, um zu einer Verbindung der Formel (IV)

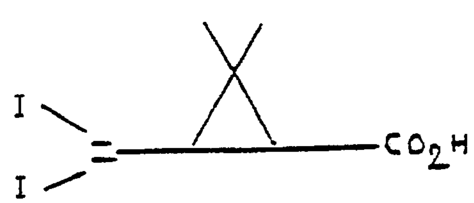

(IV)

zu gelangen, die man gegebenenfalls in ein funktionelles Derivat, wie das Säurechlorid, Säureanhydrid oder gemischte Anhydrid, überführt, die Säure der Formel (IV) oder das funktionelle Derivat dieser Säure mit dem Alkohol der Formel
R–OH
oder mit einem funktionellen Derivat dieses Alkohols umsetzt, um zu dem entsprechenden Produkt der allgemeinen Formel (I) zu gelangen:

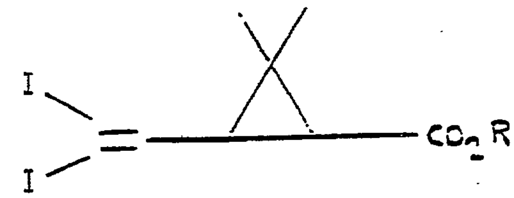

(I)

10. Verfahren gemäß Anspruch 9, dadurch gekennzeichnet, daß
das organische Lösungsmittel, in dessen Medium man das Tetrajodmethan und Triphenylphosphin mit einer Verbindung der Formel (II) umsetzt, ein chloriertes, aliphatisches Lösungsmittel, wie Methylenchlorid, ist,
das organische Lösungsmittel, in dessen Medium man die p-Toluolsulfonsäure mit einer Verbindung der Formel (III) umsetzt, ein aromatisches Lösungsmittel, wie Benzol, Toluol oder Xylol, ist
das funktionelle Derivat der Säure der Formel (IV) das Säurechlorid ist und man das Säurechlorid mit dem Alkohol R–OH in Gegenwart einer tertiären Base umsetzt,
man die Säure der Formel (IV) mit dem Alkohol R–OH in Gegenwart von Dicyclohexylcarbodiimid oder Diisopropylcarbodiimid und Dimethylaminopyridin umsetzt.

11. Verfahren gemäß Anspruch 9, dadurch gekennzeichnet, daß man die Verbindung der Formel (II)

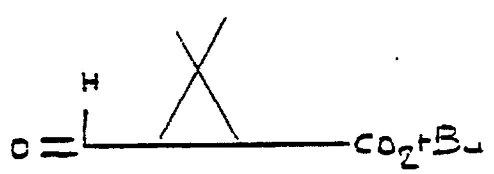

(II)

mit Tetrajodmethan, Triphenylphosphin und Zink in einem organischen Lösungsmittel umsetzt, um zu einer Verbindung der Formel (IV)

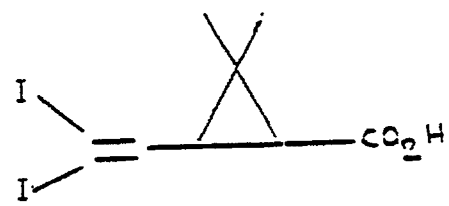

(IV)

zu gelangen, und hiernach die Synthese gemäß Anspruch 9 beendet.

12. Verfahren gemäß Anspruch 11, dadurch gekennzeichnet, daß das verwendete organische Lösungsmittel ein aliphatisches, chloriertes Lösungsmittel, wie Methylenchlorid, ist.

13. Verbindungen der Formel I gemäß einem der Ansprüche 1 bis 8 für die Verwendung bei der Bekämpfung von Parasiten der Pflanzen, Parasiten von Räumlichkeiten und Parasiten warmblütiger Tiere.

14. Pestizide Zusammensetzungen für die Bekämpfung von Parasiten der Pflanzen, Parasiten von Räumlichkeiten und Parasiten warmblütiger Tiere, dadurch gekennzeichnet, daß sie als Wirkstoff zumindest eine Verbindung gemäß einem der Ansprüche 1 bis 8 enthalten.

15. Insektizide Zusammensetzungen, enthaltend als Wirkstoff zumindest eine der Verbindungen gemäß einem der Ansprüche 1 bis 8.

16. Akarizide Zusammensetzungen für die Bekämpfung von parasitären Milben der Pflanzen, enthaltend als Wirkstoff zumindest eine der Verbindungen der allgemeinen Formel I gemäß einem der Ansprüche 1 bis 8.

17. Nematizide Zusammensetzungen, enthaltend als Wirkstoff zumindest eine der Verbindungen gemäß einem der Ansprüche 1 bis 8.

18. Akarizide Zusammensetzungen für die Bekämpfung von parasitären Milben der Tiere, enthaltend als Wirkstoff zumindest eine der Verbindungen gemäß einem der Ansprüche 1 bis 8.

19. Zusammensetzungen für die tierische Ernährung, dadurch gekennzeichnet, daß sie als Wirkstoff zumindest eine der Verbindungen gemäß einem der Ansprüche 1 bis 8 enthalten.

20. Assoziationen mit insektizider, akarizider oder nematizider Aktivität, dadurch gekennzeichnet, daß sie als Wirkstoff einesteils zumindest eine der Verbindungen der allgemeinen Formel (I) und anderenteils zumindest einen der Pyrethrinoidester, ausgewählt unter den Estern der Allethrolone, des 3,4,5,6-Tetrahydrophthalimidomethylalkohols, des 5-Benzyl-3-furylmethylalkohols, des 3-Phenoxyben-

zylalkohols und der α-Cyano-3-phenoxybenzylalkohole der Chrysanthemumsäuren, den Estern des 5-Benzyl-3-furylmethylalkohols der 2,2-Dimethyl-3-(2-oxo-3-tetrahydrothiophenylidenmethyl)-cyclopropan-1-carbonsäuren, den Estern des 3-Phenoxybenzylalkohols und der α-Cyano-3-phenoxybenzylalkohole der 2,2-Dimethyl-3-(2,2-dichlorvinyl)-cyclopropan-1-carbonsäuren, den Estern der α-Cyano-3-phenoxybenzylalkohole der 2,2-Dimethyl-3-(2,2-dibromvinyl)-cyclopropan-1-carbonsäuren, den Estern des 3-Phenoxybenzylalkohols der 2-p-Chlorphenyl-2-isopropylessigsäuren, den Estern der Allethrolone, des 3,4,5,6-Tetrahydrophthalimidomethylalkohols, des 5-Benzyl-3-furylmethylalkohols, des 3-Phenoxybenzylalkohols und der α-Cyano-3-phenoxybenzylalkohole der 2,2-Dimethyl-3-(1,2,2,2-tetrahaloethyl)-cyclopropan-1-carbonsäuren, enthalten, worin «halo» ein Fluor-, Chlor- oder Bromatom wiedergibt, mit der Maßgabe, daß die Verbindungen I in sämtlichen ihrer möglichen stereoisomeren Formen ebenso wie die sauren Verknüpfungskomponenten und die Alkohole der vorstehenden Pyrethrinoidester vorliegen können.

**Patentansprüche für den Vertragsstaat ES**

1. Verfahren zur Herstellung der Verbindungen der allgemeinen Formel (I) in sämtlichen ihrer stereoisomeren Formen oder in Form von Gemischen der Stereoisomeren

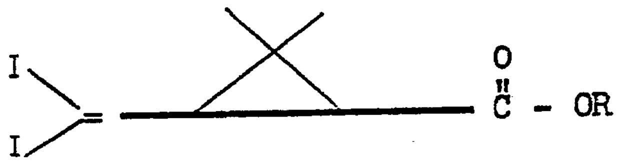

(I)

worin R den Rest eines auf dem Gebiet der Pyrethrinoide verwendeten Alkohols darstellt, dadurch gekennzeichnet, daß man sehr reines Tetrajodmethan bei einer Temperatur von etw 0°C und Triphenylphosphin in einem organischen Lösungsmittel mit einer Verbindung der Formel (II)

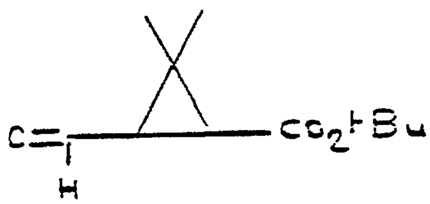

(II)

in sämtlichen ihrer stereoisomeren Formen oder in Form von Gemischen der Stereoisomeren umsetzt, die resultierende Verbindung der Formel (III)

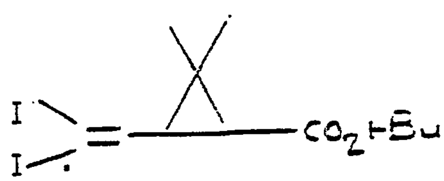

(III)

der Einwirkung von p-Toluolsulfonsäure in einem organischen Lösungsmittel unterzieht, um zu einer Verbindung der Formel (IV)

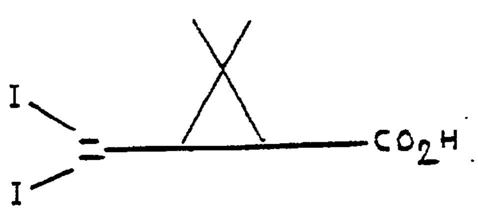

(IV)

zu gelangen, die man gegebenenfalls in ein funtionelles Derivat, wie das Säurechlorid, Säureanhydrid oder gemischte Anhydrid, überführt, die Säure der Formel (IV) oder das funktionelle Derivat dieser Säure mit dem Alkohol der Formel

R–OH

oder mit einem funktionellen Derivat dieses Alkohols umsetzt, um zu dem entsprechenden Produkt der allgemeinen Formel (I) zu gelangen:

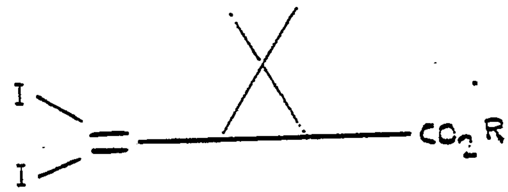

(I)

2. Verfahren gemäß Anspruch 1, dadurch gekennzeichnet, daß das organische Lösungsmittel, in dessen Medium man das Tetrajodmethan und Triphenylphosphin mit einer Verbindung der Formel (II) umsetzt, ein chloriertes, aliphatisches Lösungsmittel, wie Methylenchlorid, ist,
das organische Lösungsmittel, in dessen Medium man die p-Toluolsulfonsäure mit einer Verbindung der Formel (III) umsetzt, ein aromatisches Lösungsmittel, wie Benzol, Toluol oder Xylol, ist,
das funktionelle Derivat der Säure der Formel (IV) das Säurechlorid ist und man das Säurechlorid mit dem Alkohol R–OH in Gegenwart einer tertiären Base umsetzt,
man die Säure der Formel (IV) mit dem Alkohol ROH in Gegenwart von Dicyclohexylcarbodiimid oder Diisopropylcarbodiimid und Dimethylaminopyridin umsetzt.

3. Verfahren gemäß Anspruch 1, dadurch gekennzeichnet, daß man die Verbindung der Formel (II)

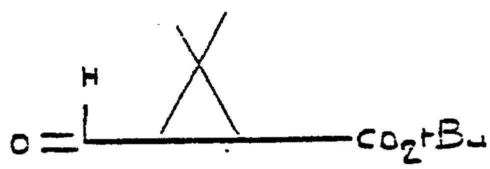

(II)

mit Tetrajodmethan, Triphenylphosphin und Zink in einem organischen Lösungsmittel umsetzt, um zu einer Verbindung der Formel (IV)

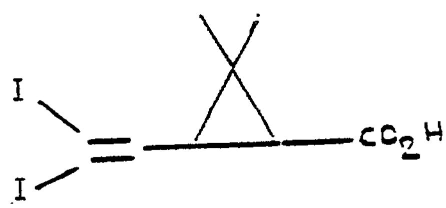

(IV)

zu gelangen, und hiernach die Synthese gemäß Anspruch 1 beendet.

4. Verfahren gemäß Anspruch 3, dadurch gekennzeichnet, daß das verwendete organische Lösungsmittel ein chloriertes, aliphatisches Lösungsmittel, wie Methylenchlorid ist.

5. Verfahren gemäß einem der Ansprüche 1 bis 4, dadurch gekennzeichnet, daß man von einem Alkohol der Formel R–OH oder einem funktionellen Derivat dieses Alkohols ausgeht, worin R bedeutet:
entweder einen linearen oder verzweigten Alkylrest mit 1 bis 8 Kohlenstoffatomen
oder einen Benzylrest
oder eine Gruppe der Formel

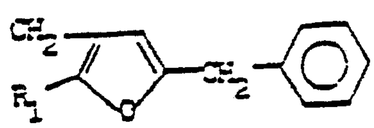

worin $R_1$ für ein Wasserstoffatom oder eine Methylgruppe steht,
oder eine Gruppe der Formel

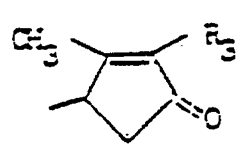

worin $R_3$ einen organischen, aliphatischen Rest mit 2 bis 6 Kohlenstoffatomen und einer oder mehreren

Kohlenstoff-Kohlenstoff-Unsättigungen und insbesondere die Reste:
$-CH_2-CH=CH_2$,
$-CH_2-CH=CH-CH_3$,
$-CH_2-CH=CH-C_2H_5$,
$-CH_2-CH=CH-CH=CH_2$,
$-CH_2-CH\equiv CH$, wiedergibt
oder eine Gruppe der Formel

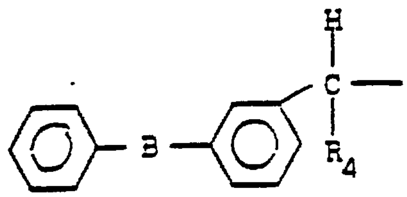

worin B eine Gruppe C=O oder ein Sauerstoffatom bedeutet und $R_4$ ein Wasserstoffatom, eine Methylgruppe, eine Gruppe $-C\equiv N$, eine Gruppe $-C\equiv CH$, eine Gruppe 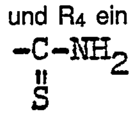 darstellt,
oder eine Gruppe der Formel

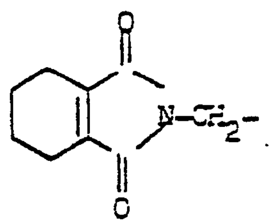

oder eine Gruppe der Formel

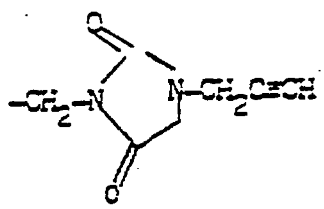

oder eine Gruppe der Formel

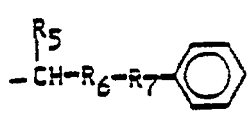

worin $R_5$ ein Wasserstoffatom oder einen Rest $-CN$ bedeutet, $R_6$ für eine Thiazolylgruppe steht, in der die Bindung 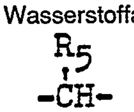 sich an irgendeiner der verfügbaren Positionen befinden kann, $R_7$ einen Rest $-CH_2-$ oder ein Sauerstoffatom darstellt,
oder eine Gruppe der Formel

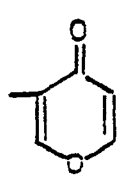

oder eine Gruppe der Formel

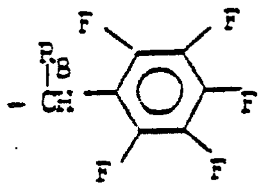

worin $R_8$ für ein Wasserstoffatom oder einen Rest CN steht,
oder eine Gruppe der Formel

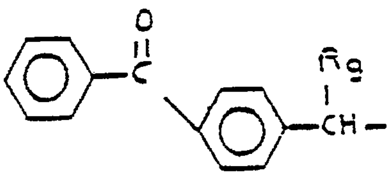

worin $R_9$ für ein Wasserstoffatom oder einen Rest –CN steht,
oder eine Gruppe der Formel

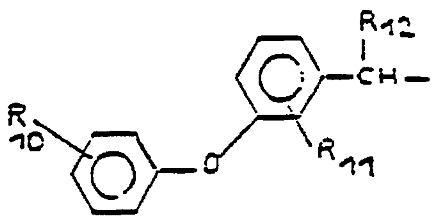

worin $R_{12}$ für ein Wasserstoffatom, eine Methyl-, Ethinyl- oder Cyanogruppe steht und $R_{10}$ und $R_{11}$, die voneinander verschieden sind, ein Wasserstoff-, Fluor-, Chlor- oder Bromatom bedeuten,
oder eine Gruppe der Formel

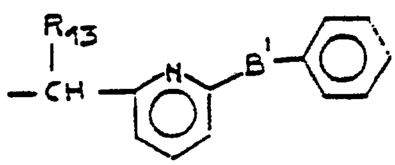

worin $R_{13}$ für ein Wasserstoffatom, eine Methyl-, Ethinyl- oder Cyanogruppe steht und B′ ein Sauerstoff- oder Schwefelatom wiedergibt,
oder eine Gruppe der Formel

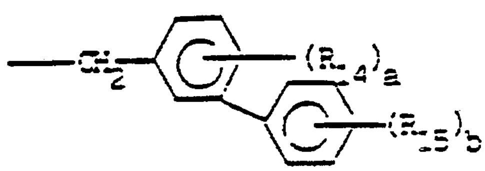

worin $R_{14}$ ein Halogenatom, eine Alkylgruppe mit 1 bis 6 Kohlenstoffatomen oder eine Trifluormethylgruppe bedeutet, $R_{15}$ für ein Halogenatom, eine Alkylgruppe mit 1 bis 6 Kohlenstoffatomen, eine Alkoxygruppe mit 1 bis 6 Kohlenstoffatomen oder eine Trifluormethylgruppe steht, a für 0, 1, 2, 3 oder 4 steht und b 0, 1, 2, 3, 4 oder 5 ist.

6. Verfahren gemäß einem der Ansprüche 1 bis 4, dadurch gekennzeichnet, daß man von einem Alkohol der Formel R–OH oder einem funktionellen Derivat dieses Alkohols ausgeht, worin R eine Gruppe der Formel

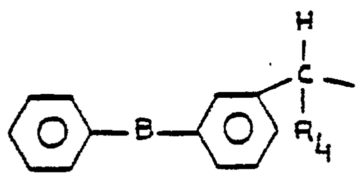

wiedergibt, in der B eine Gruppe C=O oder Sauerstoff bedeutet und $R_4$ für Wasserstoff, einen Methylrest, eine Gruppe $-C{\equiv}CH$ oder eine Gruppe 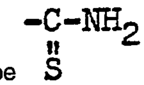 steht.

7. Verfahren gemäß einem der Ansprüche 1 bis 4, dadurch gekennzeichnet, daß man von einem Alkohol der Formel R–OH oder einem funktionellen Derivat dieses Alkohols ausgeht, worin R eine Gruppe der Formel

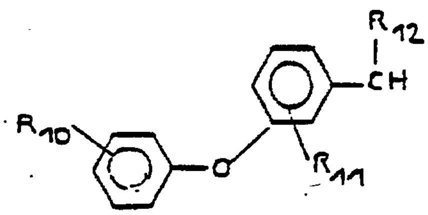

wiedergibt, in der $R_{12}$ ein Wasserstoffatom, einen Methyl-, Ethinyl- oder Cyanorest bedeutet und $R_{10}$ und $R_{11}$, die voneinander verschieden sind, für Wasserstoff oder ein Fluor-, Chlor- oder Bromatom stehen.

8. Verfahren gemäß einem der Ansprüche 1 bis 4, dadurch gekennzeichnet, daß man von einem Alkohol der Formel R–OH oder einem funktionellen Derivat dieses Alkohols ausgeht, worin R eine Gruppe der Formel

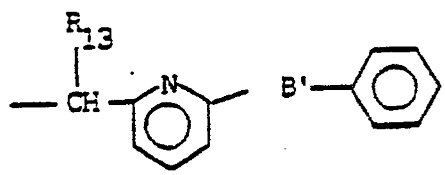

wiedergibt, in der $R_{13}$ ein Wasserstoffatom, eine Methyl-, Ethinyl- oder Cyanogruppe bedeutet und B′ für ein Sauerstoffatom oder ein Schwefelatom steht.

9. Verfahren gemäß einem der Ansprüche 1 bis 4, dadurch gekennzeichnet, daß man von einem Alkohol der Formel R–OH oder einem funktionellen Derivat dieses Alkohols ausgeht, worin R eine Gruppe der Formel

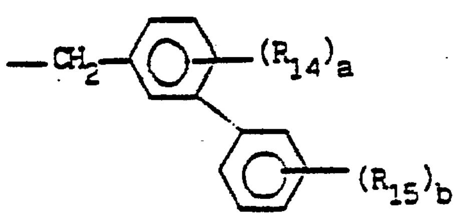

wiedergibt, worin $R_{14}$ ein Halogenatom, eine Alkylgruppe mit 1 bis 6 Kohlenstoffatomen oder eine Trifluormethylgruppe bedeutet, $R_{15}$ für ein Halogenatom, eine Alkylgruppe mit 1 bis 6 Kohlenstoffatomen, eine Alkoxygruppe mit 1 bis 6 Kohlenstoffatomen oder eine Trifluormethylgruppe steht, a 0, 1, 2, 3 oder 4 ist und b 0, 1, 2, 3, 4 oder 5 ist.

10. Verfahren gemäß einem der Ansprüche 1 bis 4, dadurch gekennzeichnet, daß man von einem Alkohol der Formel R–OH oder einem funktionellen Derivat dieses Alkohols ausgeht, worin R eine Gruppe der Formel

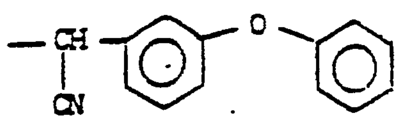 , 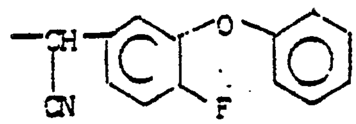

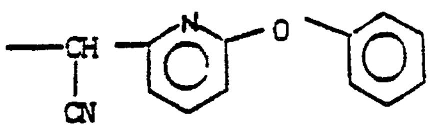 oder 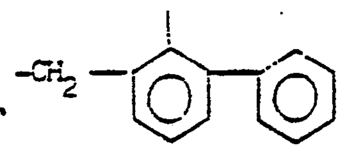

wiedergibt.

11. Verfahren gemäß einem der Ansprüche 1 bis 4, dadurch gekennzeichnet, daß man von einem Derivat der Formel (II), worin die Konfiguration 1R,cis oder 1RS,cis ist, und einem Alkohol der Formel R–OH oder einem funktionellen Derivat dieses Alkohols ausgeht, worin R eine Gruppe der Formel

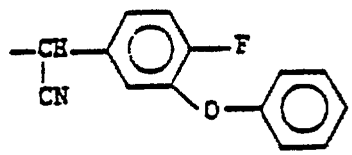

in (S)- oder (R,S)-Konfiguration wiedergibt.

**Patentansprüche für den Vertragsstaat AT**

1. Verfahren zur Herstellung der Verbindungen der allgemeinen Formel (I) in sämtlichen ihrer stereoisomeren Formen oder in Form von Gemischen der Stereoisomeren

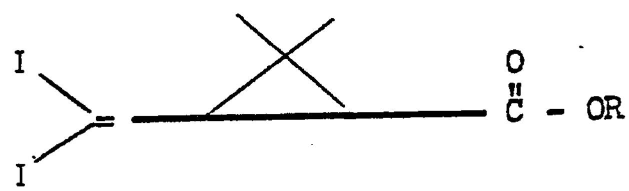

(I)

worin R den Rest eines auf dem Gebiet der Pyrethrinoide verwendeten Alkohols darstellt, dadurch gekennzeichnet, daß man sehr reines Tetrajodmethan bei einer Temperatur von etwa 0°C und Triphenylphosphin in einem organischen Lösungsmittel mit einer Verbindung der Formel (II)

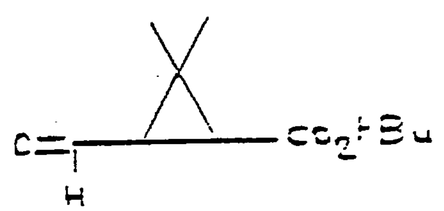

(II)

in sämtlichen ihrer stereoisomeren Formen oder in Form von Gemischen der Stereoisomeren umsetzt, die resultierende Verbindung der Formel (III)

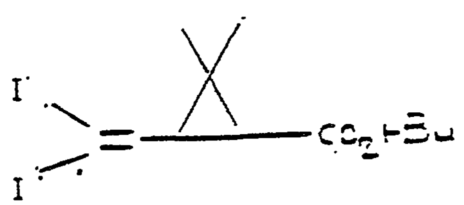

(III)

der Einwirkung von p-Toluolsulfonsäure in einem organischen Lösungsmittel unterzieht, um zu einer Verbindung der Formel (IV)

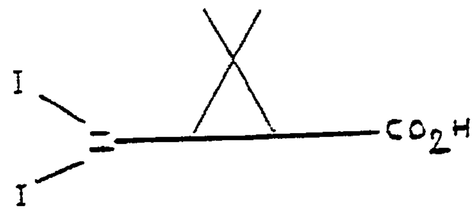

(IV)

zu gelangen, die man gegebenenfalls in ein funktionelles Derivat, wie das Säurechlorid, Säureanhydrid oder gemischte Anhydrid, überführt, die Säure der Formel (IV) oder das funktionelle Derivat dieser Säure mit dem Alkohol der Formel
R–OH
oder mit einem funktionellen Derivat dieses Alkohols umsetzt, um zu dem entsprechenden Produkt der allgemeinen Formel (I) zu gelangen:

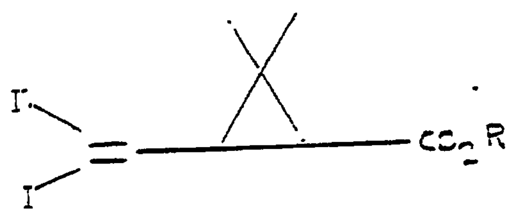

(I)

2. Verfahren gemäß Anspruch 1, dadurch gekennzeichnet, daß
das organische Lösungsmittel, in dessen Medium man das Tetrajodmethan und Triphenylphosphin mit einer Verbindung der Formel (II) umsetzt, ein chloriertes, aliphatisches Lösungsmittel, wie Methylenchlorid, ist,
das organische Lösungsmittel, in dessen Medium man die p-Toluolsulfonsäure mit einer Verbindung der Formel (III) umsetzt, ein aromatisches Lösungsmittel, wie Benzol, Toluol oder Xylol, ist
das funktionelle Derivat der Säure der Formel (IV) das Säurechlorid ist und man das Säurechlorid mit dem Alkohol R–OH in Gegenwart einer tertiären Base umsetzt,
man die Säure der Formel (VI) mit dem Alkohol ROH in Gegenwart von Dicyclohexylcarbodiimid oder Diisopropylcarbodiimid und Dimethylaminopyridin umsetzt.

3. Verfahren gemäß Anspruch 1, dadurch gekennzeichnet, daß man die Verbindung der Formel (II)

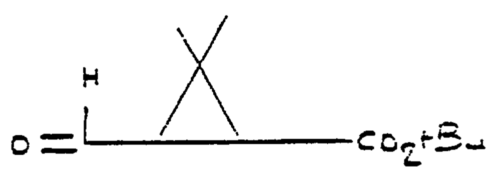

(II)

mit Tetrajodmethan, Triphenylphosphin und Zink in einem organischen Lösungsmittel umsetzt, um zu einer Verbindung der Formel (IV)

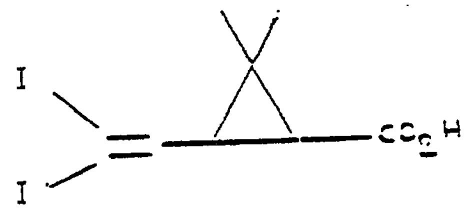

(IV)

zu gelangen, und hiernach die Synthese gemäß Anspruch 1 beendet.

4. Verfahren gemäß Anspruch 3, dadurch gekennzeichnet, daß das verwendete organische Lösungsmittel ein chloriertes, aliphatisches Lösungsmittel, wie Methylenchlorid ist.

5. Verfahren gemäß einem der Ansprüche 1 bis 4, dadurch gekennzeichnet, daß man von einem Alkohol der Formel R–OH oder einem funktionellen Derivat dieses Alkohols ausgeht, worin R bedeutet:

entweder einen linearen oder verzweigten Alkylrest mit 1 bis 8 Kohlenstoffatomen
oder einen Benzylrest
oder eine Gruppe der Formel

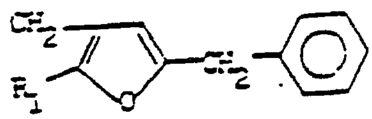

worin $R_1$ für ein Wasserstoffatom oder eine Methylgruppe steht,
oder eine Gruppe der Formel

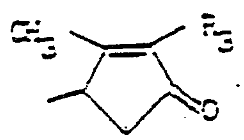

worin $R_3$ einen organischen, aliphatischen Rest mit 2 bis 6 Kohlenstoffatomen und einer oder mehreren Kohlenstoff-Kohlenstoff-Unsättigungen und insbesondere die Reste:
$-CH_2-CH=CH_2$,
$-CH_2-CH=CH-CH_3$,
$-CH_2-CH=CH-C_2H_5$,
$-CH_2-CH=CH-CH=CH_2$,
$-CH_2-C\equiv CH$, wiedergibt
oder eine Gruppe der Formel

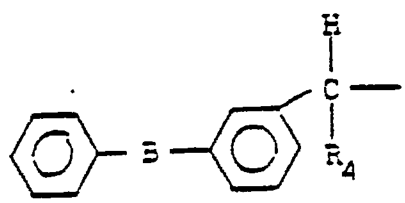

worin B eine Gruppe C=O oder ein Sauerstoffatom bedeutet und $R_4$ ein Wasserstoffatom, eine Methylgruppe, eine Gruppe $-C\equiv N$, eine Gruppe $-C\equiv CH$, eine Gruppe $-\underset{\underset{S}{\|}}{C}-NH_2$ darstellt,
oder eine Gruppe der Formel

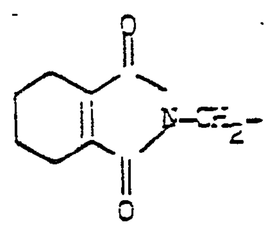

oder eine Gruppe der Formel

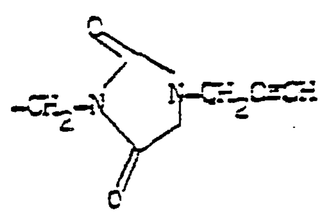

oder eine Gruppe der Formel

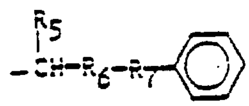

worin $R_5$ ein Wasserstoffatom oder einen Rest –CN bedeutet, $R_6$ für eine Thiazolylgruppe steht, in der die Bindung 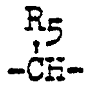 sich an irgendeiner der verfügbaren Positionen befinden kann, $R_7$ einen $-CH_2-$ oder ein Sauerstoffatom darstellt,
oder eine Gruppe der Formel

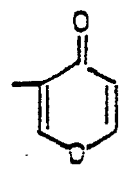

oder eine Gruppe der Formel

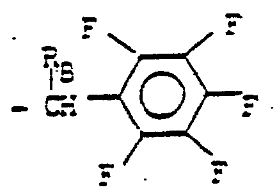

worin $R_8$ für ein Wasserstoffatom oder einen Rest CN steht,
oder eine Gruppe der Formel

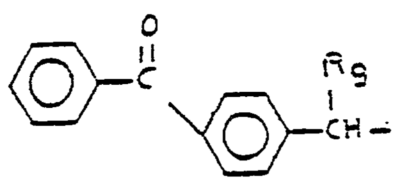

worin $R_9$ für ein Wasserstoffatom oder einen Rest –CN steht,
oder eine Gruppe der Formel

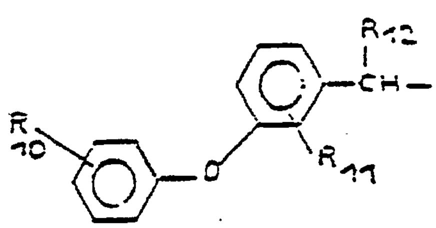

worin $R_{12}$ für ein Wasserstoffatom, eine Methyl-, Ethinyl- oder Cyanogruppe steht und $R_{10}$ und $R_{11}$, die voneinander verschieden sind, ein Wasserstoff-, Fluor-, Chlor- oder Bromatom bedeuten,
oder eine Gruppe der Formel

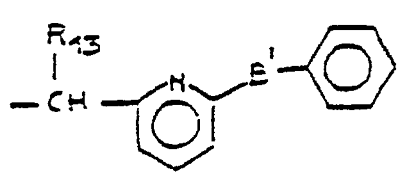

worin $R_{13}$ für ein Wasserstoffatom, eine Methyl-, Ethinyl- oder Cyanogruppe steht und B′ ein Sauerstoff- oder Schwefelatom wiedergibt,

oder eine Gruppe der Formel

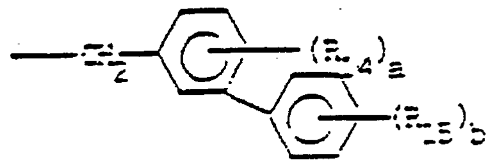

worin $R_{14}$ ein Halogenatom, eine Alkylgruppe mit 1 bis 6 Kohlenstoffatomen oder eine Trifluormethylgruppe bedeutet, $R_{15}$ für ein Halogenatom, eine Alkylgruppe mit 1 bis 6 Kohlenstoffatomen, eine Alkoxygruppe mit 1 bis 6 Kohlenstoffatomen oder eine Trifluormethylgruppe steht, a für 0, 1, 2, 3 oder 4 steht und b 0, 1, 2, 3, 4 oder 5 ist.

6. Verfahren gemäß einem der Ansprüche 1 bis 4, dadurch gekennzeichnet, daß man von einem Alkohol der Formel R–OH oder einem funktionellen Derivat dieses Alkohols ausgeht, worin R ein Gruppe der Formel

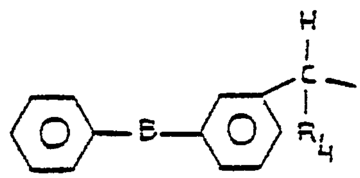

wiedergibt, in der B eine Gruppe C=O oder Sauerstoff bedeutet und $R_4$ für Wasserstoff, einen Methylrest, eine Gruppe C≡N, eine Gruppe –C≡CH oder eine Gruppe 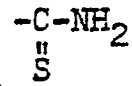 steht.

7. Verfahren gemäß einem der Ansprüche 1 bis 4, dadurch gekennzeichnet, daß man von einem Alkohol der Formel R–OH oder einem funktionellen Derivat dieses Alkohols ausgeht, worin R eine Gruppe der Formel

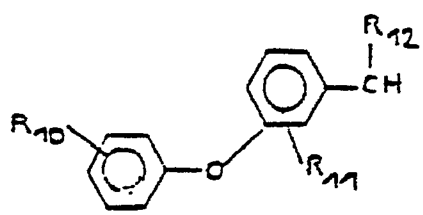

wiedergibt, in der $R_{12}$ ein Wasserstoffatom, einen Methyl-, Ethinyl- oder Cyanorest bedeutet und $R_{10}$ und $R_{11}$, die voneinander verschieden sind, für Wasserstoff oder ein Fluor-, Chlor- oder Bromatom stehen.

8. Verfahren gemäß einem der Ansprüche 1 bis 4, dadurch gekennzeichnet, daß man von einem Alkohol der Formel R–OH oder einem funktionellen Derivat dieses Alkohols ausgeht, worin R eine Gruppe der Formel

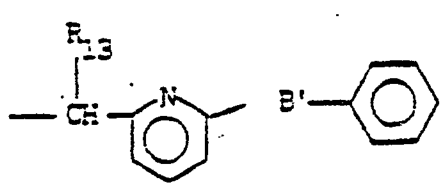

wiedergibt, in der $R_{13}$ ein Wasserstoffatom, eine Methyl-, Ethinyl- oder Cyanogruppe bedeutet und B′ für ein Sauerstoffatom oder ein Schwefelatom steht.

9. Verfahren gemäß einem der Ansprüche 1 bis 4, dadurch gekennzeichnet, daß man von einem Alkohol der Formel R–OH oder einem funktionellen Derivat dieses Alkohols ausgeht, worin R eine Gruppe der Formel

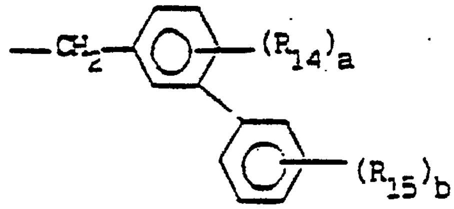

wiedergibt, worin $R_{14}$ ein Halogenatom, eine Alkylgruppe mit 1 bis 6 Kohlenstoffatomen oder eine Trifluormethylgruppe bedeutet, $R_{15}$ für ein Halogenatom, eine Alkylgruppe mit 1 bis 6 Kohlenstoffatomen, eine Alkoxygruppe mit 1 bis 6 Kohlenstoffatomen oder eine Trifluormethylgruppe steht, a 0, 1, 2, 3 oder 4 ist und b 0, 1, 2, 3, 4 oder 5 ist.

10. Verfahren gemäß einem der Ansprüche 1 bis 4, dadurch gekennzeichnet, daß man von einem Alkohol der Formel R–OH oder einem funktionellen Derivat dieses Alkohols ausgeht, worin R eine Gruppe der Formel

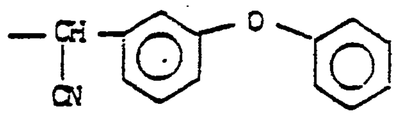 , 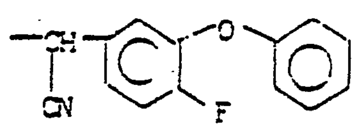

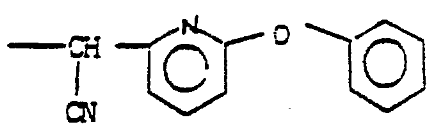 oder 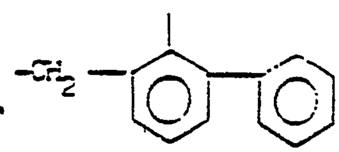

wiedergibt.

11. Verfahren gemäß einem der Ansprüche 1 bis 4, dadurch gekennzeichnet, daß man von einem Derivat der Formel (II), worin die Konfiguration 1R,cis oder 1RS,cis ist, und von einem Alkohol der Formel R–OH oder einem funktionellen Derivat dieses Alkohols ausgeht, worin R eine Gruppe der Formel

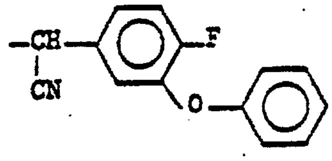

in (S)- oder (R,S)-Konfiguration wiedergibt.

12. Pestizide Zusammensetzungen für die Bekämpfung von Parasiten der Pflanzen, Parasiten von Räumlichkeiten und Parasiten warmblütiger Tiere, dadurch gekennzeichnet, daß sie als Wirkstoff zumindest eine Verbindung gemäß einem der Ansprüche 1 bis 11 enthalten.

13. Insektizide Zusammensetzungen, enthaltend als Wirkstoff zumindest eine der Verbindungen gemäß einem der Ansprüche 1 bis 11.

14. Akarizide Zusammensetzungen für die Bekämpfung von parasitären Milben der Pflanzen, enthaltend als Wirkstoff zumindest eine der Verbindungen der allgemeinen Formel I gemäß einem der Ansprüche 1 bis 11.

15. Nematizide Zusammensetzungen, enthaltend als Wirkstoff zumindest eine der Verbindungen gemäß einem der Ansprüche 1 bis 11.

16. Akarizide Zusammensetzungen für die Bekämpfung von parasitären Milben der Tiere, enthaltend als Wirkstoff zumindest eine der Verbindungen gemäß einem der Ansprüche 1 bis 11.

17. Zusammensetzungen für die tierische Ernährung, dadurch gekennzeichnet, daß sie als Wirkstoff zumindest eine der Verbindungen gemäß einem der Ansprüche 1 bis 11 enthalten.

18. Zusammensetzungen mit insektizider, akarizider oder nematizider Aktivität, dadurch gekennzeichnet, daß sie als Wirkstoff einesteils zumindest eine der Verbindungen der allgemeinen Formel (I) anderenteils zumindest einen der Pyrethrinoidester, ausgewählt unter den Estern der Allethrolone, des 3,4,5,6-Tetrahydrophthalimidomethylalkohols, des 5-Benzyl-3-furylmethylalkohols, des 3-Phenoxybenzylalkohols und der α-Cyano-3-phenoxybenzylalkohole der Chrysanthemumsäuren, den Estern des 5-Benzyl-3-furylmethylalkohols der 2,2-Dimethyl-3-(2-oxo-3-tetrahydrothiophenylidenmethyl)-cyclopro-

pan-1-carbonsäuren, den Estern des 3-Phenoxybenzylalkohols und der α-Cyano-3-phenoxybenzylalkohole der 2,2-Dimethyl-3-(2,2-dichlorvinyl)-cyclopropan-1-carbonsäuren, den Estern der α-Cyano-3-phenoxybenzylalkohole der 2,2-Dimethyl-3-(2,2-dibromvinyl)-cyclopropan-1-carbonsäuren, den Estern des 3-Phenoxybenzylalkohols der 2-p-Chlorphenyl-2-isopropylessigsäuren, den Estern der Allethrolone, des 3,4,5,6-Tetrahydrophthalimidomethylalkohols, des 5-Benzyl-3-furylmethylalkohols, des 3-Phenoxybenzylalkohols und der α-Cyano-3-phenoxybenzylalkohole der 2,2-Dimethyl-3-(1,2,2,2-tetrahaloethyl)-cyclopropan-1-carbonsäuren, enthalten, worin «halo» ein Fluor-, Chlor- oder Bromatom wiedergibt, mit der Maßgabe, daß die Verbindungen I in sämtlichen ihrer möglichen stereoisomeren Formen ebenso wie die sauren Verknüpfungskomponenten und die Alkohole der vorstehenden Pyrethrinoidester vorliegen können.

**Claims for the contracting States BE, CH, DE, FR, GB, IT, LU, NL**

1. The compounds of general formula (I) in all their stereoisomer forms or in the form of a mixture of stereoisomers:

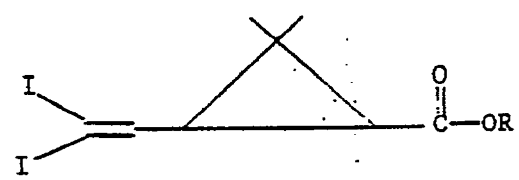

(I)

in which R represents the remainder of an alcohol used in the domain of pyrethrinoids.

2. The compounds of general formula (I), according to claim 1, in all their stereoisomer forms or in the form of a mixture of stereoisomers, characterised in that R represents:

– either a linear or branched alkyl radical, containing from 1 to 8 carbon atoms,

– or a benzyl radical,

– or a group of formula:

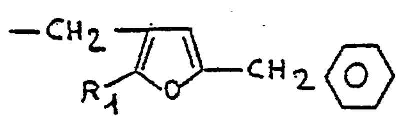

in which $R_1$ represents a hydrogen atom or a methyl radical, or a group of formula:

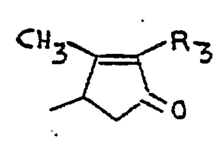

in which $R_3$ represents an organic aliphatic radical containing from 2 to 6 carbon atoms and one or more carbon-carbon unsaturations and more particularly the radicals $-CH_2=CH=CH_2$, $-CH_2-CH=CH-CH_3$, $-CH_2-CH=CH-C_2H_5$, $-CH_2-CH=CH-CH=CH_2$, $CH_2-C=CH$,

– or a group of formula:

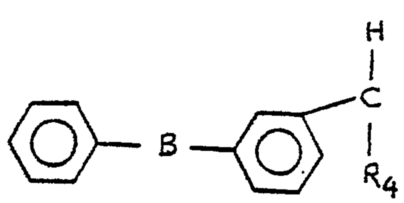

in which B represents a C=O group or an oxygen atom and $R_4$ represents a hydrogen atom, a methyl radical, a –C≡N group, a –C≡CH group, a 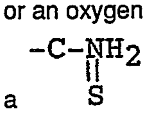 group,

– or a group of formula:

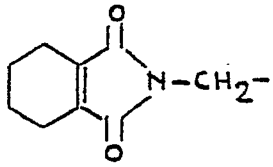

– or a group of formula:

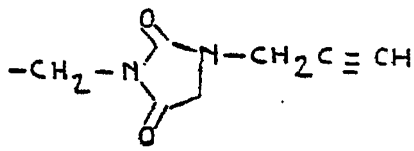

– or a group of formula:

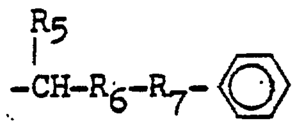

in which $R_5$ represents a hydrogen atom or a –CN radical, $R_6$ represents a thiazolyl radical in which the 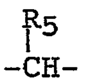 liaison can be at any one of the available positions, $R_7$ represents a $-CH_2-$ radical or an oxygen atom:
– or a group of formula:

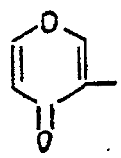

– or a group of formula:

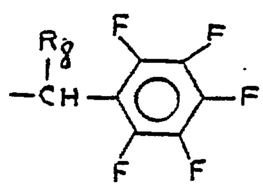

in which $R_8$ represents a hydrogen atom or a CN radical,
– or a group of formula:

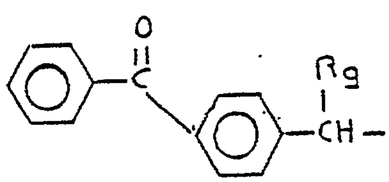

in which $R_9$ represents a hydrogen atom or a –CN radical,
– or a group of formula:

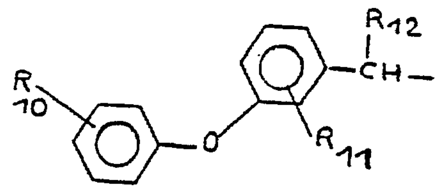

in which $R_{12}$ represents a hydrogen atom, a methyl, ethynyl or cyano radical and $R_{10}$ and $R_{11}$ different from one another represent a hydrogen, fluorine, chlorine or bromine atom,
– or a group of formula:

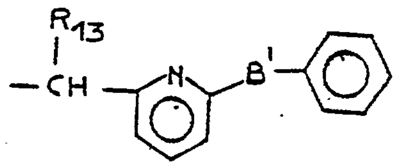

in which $R_{13}$ represents a hydrogen atom, a methyl, ethynyl or cyano radical and B′ represents an oxygen or sulphur atom,
– or a group of formula:

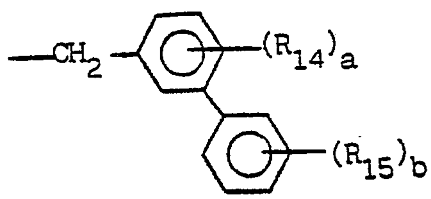

in which $R_{14}$ represents a halogen atom, an alkyl radical containing from 1 to 6 carbon atoms or a trifluoromethyl radical, $R_{15}$ represents a halogen atom, an alkyl radical containing from 1 to 6 carbon atoms, an alkoxy radical containing from 1 to 6 carbon atoms or a trifluoromethyl radical, a is 0,1, 2, 3 or 4 and b is 0, 1, 2, 3, 4, or 5.

3. The compounds of general formula (I) according to claims 1 or 2 in all their stereoisomer forms or in the form of a mixture of stereoisomers, characteriesed in that R represents a group of formula:

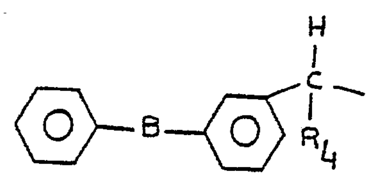

in which B represents a C=O group or oxygen and $R_4$ represents hydrogen, a methyl radical, a C≡N group, a –C≡CH group or a 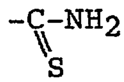 group.

4. The compounds of general formula (I), according to claims 1 or 2, in all their stereoisomer forms or in the form of a mixture of stereoisomers, characterised in that R represents a group of formula:

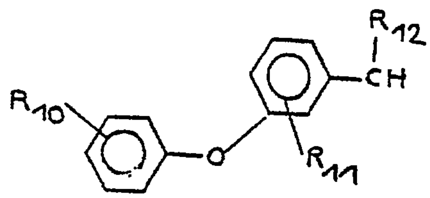

in which $R_{12}$ represents a hydrogen atom, a methyl, ethynyl or cyano radical, and $R_{10}$ and $R_{11}$ different to one another represent hydrogen or a fluorine, chlorine or bromine atom.

5. The compounds of general formula (I) according to claim 1 or 2 in all their stereoisomer forms or in the form of a mixture of stereoisomers, characterised in that R represents a group of formula:

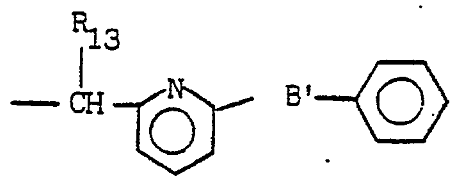

in which $R_{13}$ represents a hydrogen atom, a methyl, ethynyl or cyano radical and B′ represents an oxygen or sulphur atom.

6. The compounds of general formula (I) according to claims 1 or 2 in all their stereoisomer forms or in the form of a mixture of stereoisomers, characterised in that R represents a group of formula:

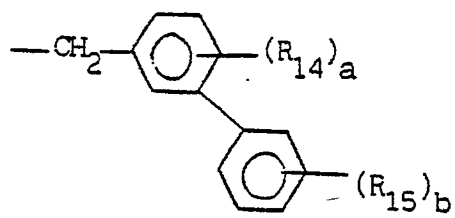

in which $R_{14}$ represents a halogen atom, an alkyl radical containing from 1 to 6 carbon atoms or a trifluoromethyl radical, $R_{15}$ represents a halogen atom, an alkyl radical containing from 1 to 6 carbon atoms, an alkoxy radical containing from 1 to 6 carbon atoms or a trifluoromethyl radical, a is 0, 1, 2, 3 or 4 and b is 0, 1, 2, 3, 4 or 5.

7. The compounds of general formula (I) in all their stereoisomer forms or in the form of a mixture of stereoisomers, according to any one of claims 1 to 6, characterised in that R represents a group of formula:

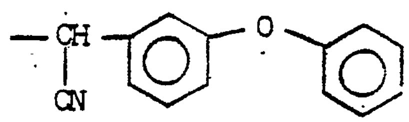
,
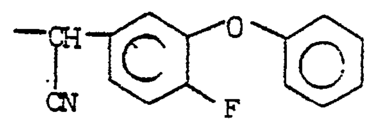

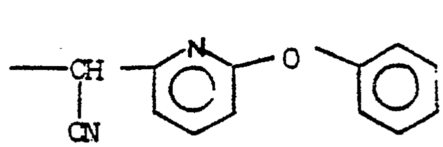
or
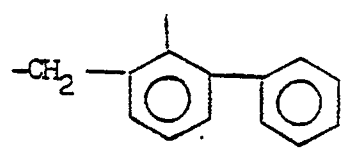

8. The compounds the names of which follow:
- (S)-cyano-(3-phenoxy-4-fluoro-phenyl)-methyl 1R,cis-2,2-dimethyl-3-[2,2-diiodo-ethenyl]-cyclopropane carboxylate,
- (S)-cyano-1-(3-phenoxy-4-fluorophenyl)-methyl 1R,cis-2,2-dimethyl-3-(2,2-diiodoethenyl)-cyclopropane carboxylate and (R)-cyano-1-(3-phenoxy-4-fluorophenyl)-methyl 1S,cis-2,2-dimethyl-3-(2,2-diiodoethenyl)-cyclopropane carboxylate in a 50–50 mixture.

9. Preparation process of compounds of general formula (I), as defined in claim 1, in all of their stereoisomer forms or in the form of a mixture of stereoisomers, characterised in that very pure tetraiodomethane at a temperature of approximately 0°C and triphenylphosphine are reacted, in an organic solvent, with a compound of formula (II):

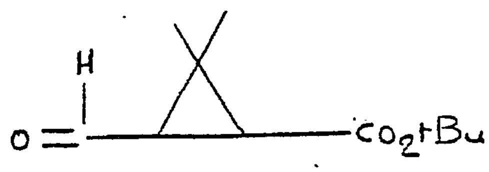

(II)

in all its stereoisomer forms or in the form of a mixture of stereoisomers, the resultant compound of formula (III):

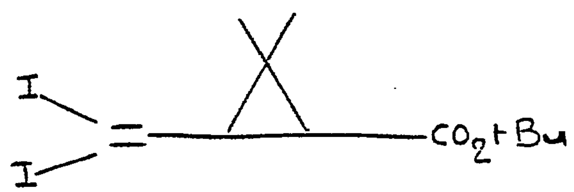

(III)

is subjected to the action of paratoluene sulphonic acid in an organic solvent so as to obtain a compound of formula (IV):

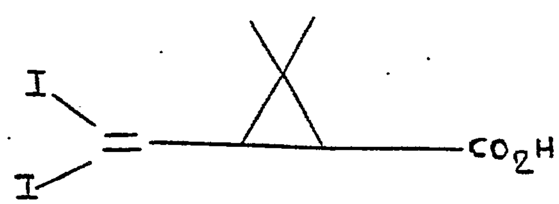

(IV)

which is optionally converted into a functional derivative such as the acid chloride, anhydride or mixed anhydride, the acid of formula (IV) or its functional derivative is reacted with an alcohol of formula:
R–OH
or with a functional derivative of this alcohol so as to obtain the corresponding product of formula (I):

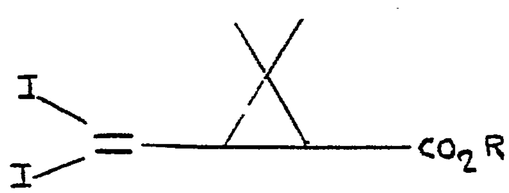

(I)

10. Preparation process according to claim 9 characterised in that
– the organic solvent in which the tetraiodomethane and the triphenyl-phosphine are reacted with compound of formula (II) is a chlorinated aliphatic solvent such as methylene chloride,
– the organic solvent in which the paratoluene sulphonic acid is reacted with a compound of formula (III) is an aromatic solvent such as benzene, toluene or xylene, the functional derivative of the acid of formula (IV) is the acid chloride and the acid chloride is reacted with the alcohol R–OH in the presence of a tertiary base,
– the acid of formula (IV) is reacted with the alcohol ROH, in the presence of dicyclohexyl carbodiimide or diisopropyl carbodiimide and dimethyl amino pyridine.

11. Preparation process according to claim 9, characterised in that the compound of formula (II):

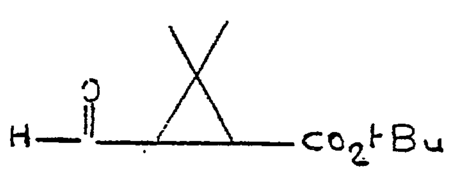

(II)

is reacted with tetraiodomethane, triphenylphosphine and zinc in an organic solvent so as to obtain a compound of formula (IV):

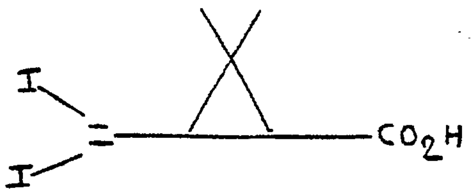

(IV)

then the synthesis is completed as in claim 9.

12. Preparation process according to claim 11, characterised in that the organic solvent used is a chlorinated aliphatic solvent such as methylene chloride.

13. The compounds of formula I as defined in any one of claims 1 to 8, for their use in combating parasites of vegetation, parasites of premises and parasites of warmblooded animals.

14. The pesticide compositions intended to combat parasites of vegetation, parasites of premises and parasites of warmblooded animals, characterised in that they contain as active principle at least one compound defined in any one of claims 1 to 8.

15. The insecticide compositions containing as active principle at least one of the compounds defined in any one of claims 1 to 8.

16. The acaricide compositions intended to combat acaride parasites of vegetation, containing as active principle at least one of the compounds of general formula (I), as defined in any one of claims 1 to 8.

17. The nematicide compositions containing as active principle at least one of the compounds defined in any one of claims 1 to 8.

18. The acaricide compositions intended to combat the acaride parasites of animals, containing as active principle at least one of the compounds defined in any one of claims 1 to 8.

19. The compositions intended for animal fodder, characterised in that they contain as active principle at least one of the compounds as defined in any one of claims 1 to 8.

20. Combinations endowed with insecticide, acaricide or nematicide activity, characterised in that they contain as active principle, on the one hand, at least one of the compounds of general formula (I), and on the other hand, at least one of the pyrethrinoid esters chosen from the group constituted by the esters of allethrolone, of 3,4,5,6-tetrahydro-phtalimidomethyl alcohol, of 5-benzyl-3-furyl methyl alcohol, of 3-phenoxy-benzyl alcohol, and of alpha-cyano-3-phenoxybenzyl alcohols with chrysanthemic acids, by the esters of 5-benzyl-3-furyl methyl alcohols with 2,2-dimethyl-3-(2-oxo-3-tetrahydrothiophenylidenemethyl)-cyclopropane-1-carboxylic acids, by the esters of 3-phenoxybenzyl alcohols and of alpha-cyano-3-phenoxybenzyl alcohols with 2,2-dimethyl-3-(2,2-dichlorovinyl)-cyclopropane-1-carboxylic acids, by the esters of alpha-cyano-3-phenoxy-benzyl alcohols with 2,2-dimethyl-3-(2,2-dibromovinyl)-cyclopropane-1-carboxylic acids, by the esters of 3-phenoxybenzyl alcohols with 2-parachlorophenyl-2-isopropyl acetic acids, by the esters of allethrolone, of 3,4,5,6-tetrahydrophtal imidomethyl alcohol, of 5-benzyl-3-furyl methyl alcohol, of 3-phenoxybenzyl alcohol, and of alpha-cyano-3-phenoxybenzyl alcohols with 2,2-dimethyl-3-(1,2,2,2,-tetrahaloethyl)-cyclopropane-1-carboxylic acids, in which "halo" represents a fluorine, chlorine or bromine atom, it being understood that the compounds (I) can exist in all their possible stereoisomer forms, the same being true for the acid and alcohol copulas of the above pyrethrinoid esters.

**Claims for the contracting State AT**

1. Preparation process of compounds of general formula (I), in all of their stereoisomer forms or in the form of a mixture of stereoisomers:

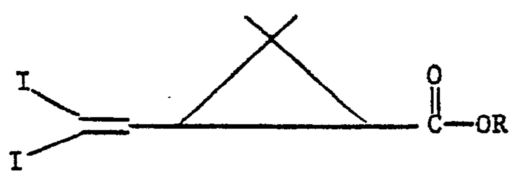

(I)

in which R represents the remainder of an alcohol used in the domain of pyrethrinoids, characterised in that very pure tetraiodomethane, at a temperature of approximately 0°C and triphenylphosphine are reacted, in an organic solvent, with a compound of formula (II):

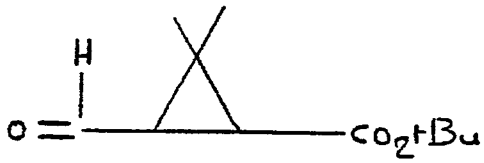

(II)

in all its stereoisomer forms or in the form of a mixture of stereoisomers, the resultant compound of formula (III):

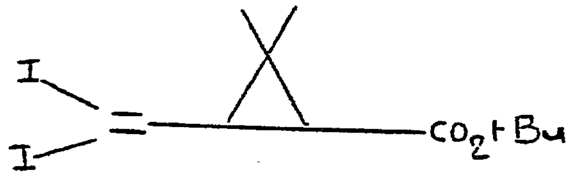

(III)

is subjected to the action of paratoluene sulphonic acid in an organic solvent so as to obtain a compound of formula (IV):

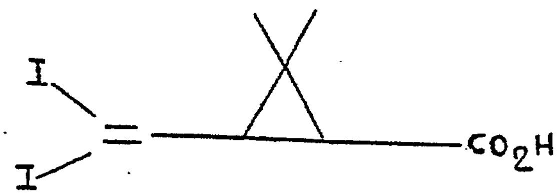

(IV)

which is optionally converted into a functional derivative such as the acid chloride, anhydride or mixed anhydride, the acid of formula (IV) or its functional derivative is reacted with an alcohol of formula:
R–OH
or with a functional derivative of this alcohol so as to obtain the corresponding product of general formula (I):

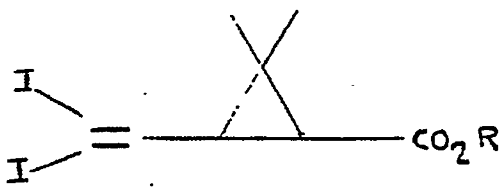

(I)

2. Preparation process according to claim 1 characterised in that
– the organic solvent in which the tetraiodomethane and the triphenyl-phosphine are reacted with a compound of formula (II) is a chlorinated aliphatic solvent such as methylene chloride,
– the organic solvent in which the paratoluene sulphonic acid is reacted with a compound of formula (III) is an aromatic solvent such as benzene, toluene or xylene, the functional derivative of the acid of formula (IV) is the acid chloride and the acid chloride is reacted with the alcohol R–OH in the presence of a tertiary base,
– the acid of formula (IV) is reacted with the alcohol ROH, in the presence of dicyclohexyl carbodiimide or diisopropyl carbodiimide and dimethyl amino pyridine.

3. Preparation process according to claim 1, characterised in that the compound of formula (II):

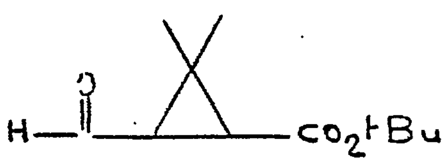

(II)

is reacted with tetraiodomethane, triphenylphosphine and zinc in an organic solvent so as to obtain a compound of formula (IV):

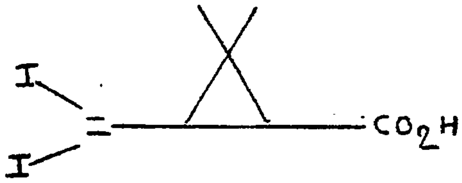

(IV)

then the synthesis is completed as in claim 1.

4. Preparation process according to claim 3, characterised in that the organic solvent used is a chlorinated aliphatic solvent such as methylene chloride.

5. Process according to any one of claims 1 to 4, characterised in that to start with an alcohol of formula R–OH or a functional derivative of this alcohol is used, formula in which R represents:
– either a linear or branched alkyl radical, containing from 1 to 8 carbon atoms,
– or a benzyl radical,
– or a group of formula:

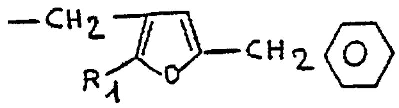

in which $R_1$ represents a hydrogen atom or a methyl radical, or a group of formula:

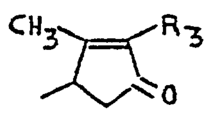

in which $R_3$ represents an organic aliphatic radical containing from 2 to 6 carbon atoms and one or more carbon-carbon unsaturations and more particularly the radicals
$-CH_2=CH=CH_2$, $-CH_2-CH=CH-CH_3$,
$-CH_2-CH=CH-C_2H_5$, $-CH_2-CH=CH-CH=CH_2$, $CH_2-C\equiv CH$,
– or a group of formula:

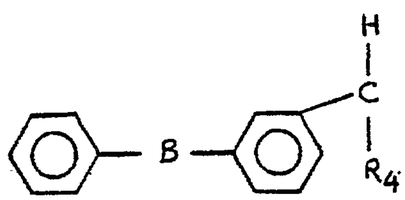

in which B represents a C = O group or an oxygen atom and $R_4$ represents a hydrogen atom, a methyl radical, a $-C\equiv N$ group, $-C\equiv CH$ group, a 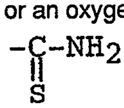 group,
– or a group of formula:

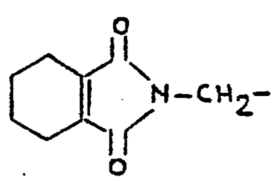

– or a group of formula:

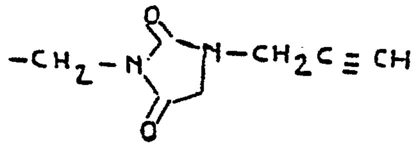

– or a group of formula:

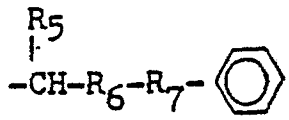

in which $R_5$ represents a hydrogen atom or a –CN radical, $R_6$ represents a thiazolyl radical in which the 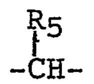 bond can be at any one of the available positions, $R_7$ represents a $-CH_2-$ radical or an oxygen atom:

– or a group of formula:

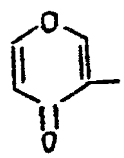

– or a group of formula:

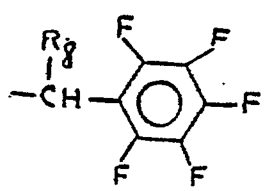

in which $R_8$ represents a hydrogen atom or a CN radical,

– or a group of formula:

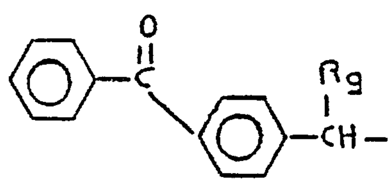

in which $R_9$ represents a hydrogen atom or a –CN radical,

– or a group of formula:

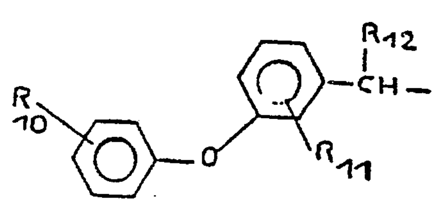

in which $R_{12}$ represents a hydrogen atom, a methyl, ethynyl or cyano radical and $R_{10}$ and $R_{11}$ different from one another represent a hydrogen, fluorine, chlorine or bromine atom,

– or a group of formula:

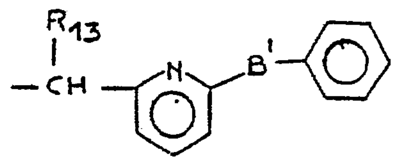

in which $R_{13}$ represents a hydrogen atom, a methyl, ethynyl or cyano radical and B′ represents an oxygen or sulphur atom,

– or a group of formula:

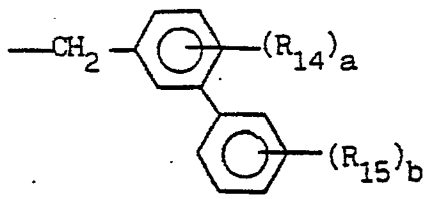

in which $R_{14}$ represents a halogen atom, an alkyl radical containing from 1 to 6 carbon atoms or a trifluoromethyl radical, $R_{15}$ represents a halogen atom, an alkyl radical containing from 1 to 6 carbon atoms, an alkoxy radical containing from 1 to 6 carbon atoms or a trifluoromethyl radical, a is 0, 1, 2, 3 or 4 and b is 0, 1, 2, 3, 4, or 5.

6. Process according to any one of claims 1 to 4, characterised in that to start with an alcohol of formula R–OH or a functional derivative of this alcohol is used, formula in which R represents a group of formula:

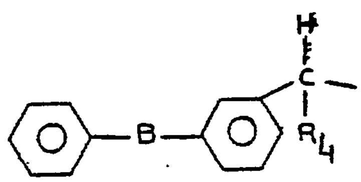

in which B represents a C = O group or oxygen and $R_4$ represents hydrogen, a methyl radical, a C ≡ N group, a C ≡ CH group or a 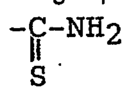 group.

7. Process according to any one of claims 1 to 4, characterised in that to start with an alcohol of formula R–OH or a functional derivative of this alcohol is used, formula in which R represents a group of formula:

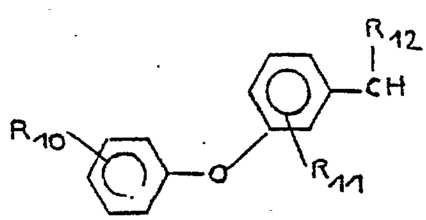

in which $R_{12}$ represents a hydrogen atom, a methyl, ethynyl or cyano radical, and $R_{10}$ and $R_{11}$ different to one another represent hydrogen or a fluorine, chlorine or bromine atom.

8. Process according to any one of claims 1 to 4, characterised in that to start with an alcohol of formula R–OH or a functional derivative of this alcohol is used, formula in which R represents a group of formula:

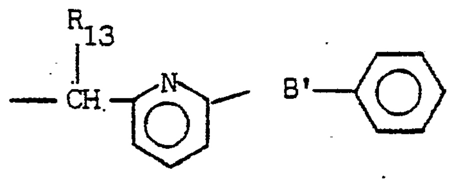

in which $R_{13}$ represents a hydrogen atom, a methyl, ethynyl or cyano radical and B′ represents an oxygen or sulphur atom.

9. Process according to any one of claims 1 to 4, characterised in that to start with an alcohol of formula R–OH or a functional derivative of this alcohol is used, formula in which R represents a group of formula:

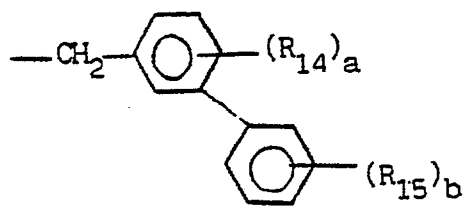

in which $R_{14}$ represents a halogen atom, an alkyl radical containing from 1 to 6 carbon atoms or a trifluoromethyl radical, $R_{15}$ represents a halogen atom, an alkyl radical containing from 1 to 6 carbon atoms, an alkoxy radical containing from 1 to 6 carbon atoms or a trifluoromethyl radical, a is 0, 1, 2, 3 or 4 and b is 0, 1, 2, 3, 4 or 5.

10. Process according to any one of claims 1 to 4, characterised in that to start with an alcohol of formula R–OH or a functional derivative of this alcohol is used, formula in which R represents a group of formula:

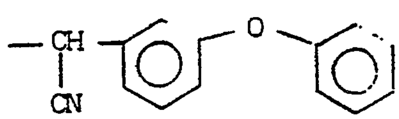 , 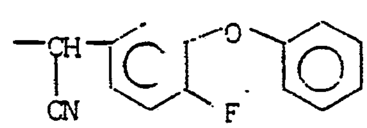

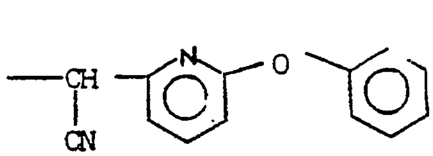 or 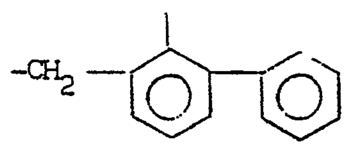

11. Process according to any one of claims 1 to 4, characterised in that to start with a derivative of formula (II) is used in which the configuration is 1R, cis or 1RS, cis and an alcohol of formula R–OH, or a functional derivative of this alcohol, formula in which R represents a group of formula:

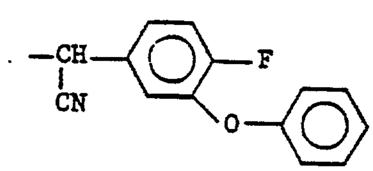

of configuration (S) or (R, S).

12. The pesticide compositions intended to combat parasites of vegetation, parasites of premises and parasites of warmblooded animals, characterised in that they contain as active principle at least one compound defined in any one of claims 1 to 11.

13. The insecticide compositions containing as active principle, at least one of the compounds defined in any one of claims 1 to 11.

14. The acaricide compositions intended to combat acaride parasites of vegetation, containing as active principle at least one of the compounds of general formula (I), as defined in any one of claims 1 to 11.

15. The nematicide compositions containing as active principle at least one of the compounds defined in any one of claims 1 to 11.

16. The acaricide compositions intended to combat the acaride parasites of animals, containing as active principle at least one of the compounds defined in any one of claims 1 to 11.

17. The compositions intended for animal fodder, characterised in that they contain as active principle at least one of the compounds as defined in any one of claims 1 to 11.

18. Combinations endowed with insecticide, acaricide or nematicide activity, characterised in that they contain as active principle, on the one hand, at least one of the compounds of general formula (I), and on the other hand, at least one of the pyrethrinoid esters chosen from the group constituted by the esters of allethrolone, of 3, 4, 5, 6- tetrahydro-phtalimido-methyl alcohol, of 5-benzyl-3-furyl methyl alcohol, of 3-phenoxybenzyl alcohol, and of alphacyano-3-phenoxybenzyl alcohols with chrysanthemic acids, by the esters of 5-benzyl-3-furyl methyl alcohols with 2,2-dimethyl-3-(2-oxo-3-tetrahydrothiophenylidene-methyl)-cyclopropane-1-carboxylic acids, by the esters of 3-phenoxybenzyl alcohols and of alpha-cyano-3-phenoxybenzyl alcohols with 2,2-dimethyl-3-(2,2-dichlorovinyl)-cyclopropane-1-carboxylic acids, by the esters of alphacyano-3-phenoxy-benzyl alcohols with 2,2-dimethyl-3-(2,2-dibromovinyl)-cyclopropane-1-carboxylic acids, by the esters of 3-phenoxybenzyl alcohols with 2-parachlorophenyl-2-isopropyl acetic acids, by the esters of allethrolone, of 3, 4, 5, 6-tetrahydrophtal-imidomethyl alcohol, of 5-benzyl-3-furyl methyl alcohol, of 3-phenoxy benzyl alcohol, and of alpha-cyano-3-phenoxy benzyl alcohols with 2,2-dimethyl-3-(1, 2, 2, 2, -tetrahaloethyl)-cyclopropane-1-carboxylic acids, in which "halo" represents a fluorine, chlorine or bromine atom, it being understood that the compounds (I) can exist in all their possible stereoisomer forms, the same being true for the acid and alcohol copulas of the above pyrethrinoid esters.

**Claims for the contracting State ES**

1. Preparation process of compounds of general formula (I) in all of their stereoisomer forms or in the form of a mixture of stereoisomers:

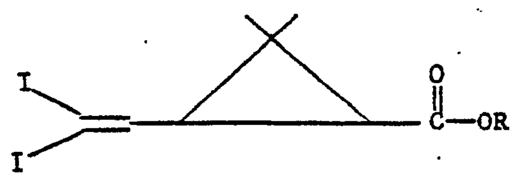

(I)

in which R represents the remainder of an alcohol used in the domain of pyrethrinoids, characterised in that very pure tetraiodomethane, at a temperature of approximately 0°C and triphenylphosphine are reacted, in an organic solvent, with a compound of formula (II):

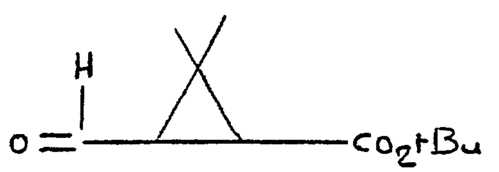

(II)

in all its stereoisomer forms or in the form of a mixture of stereoisomers, the resultant compound of formula (III):

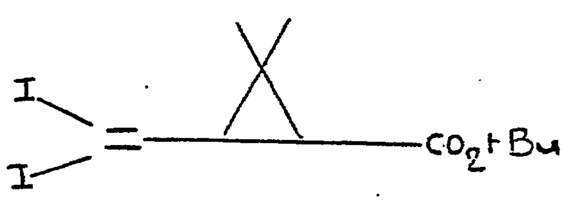

(III)

is subjected to the action of paratoluene sulphonic acid in an organic solvent so as to obtain a compound of formula (IV):

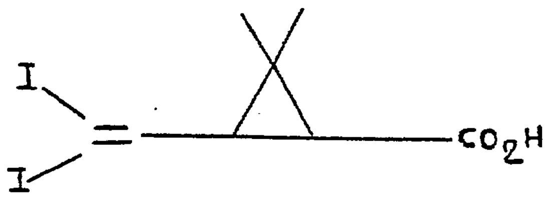

(IV)

which is optionally converted into a functional derivative such as the acid chloride, anhydride or mixed anhydride, the acid of formula (IV) or its functional derivative is reacted with an alcohol of formula:
R–OH
or with a functional derivative of this alcohol so as to obtain the corresponding product of general formula (I):

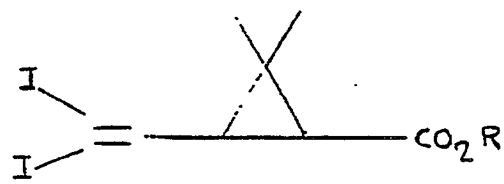

(I)

2. Preparation process according to claim 1 characterised in that
– the organic solvent in which the tetraiodomethane and the triphenyl-phosphine are reacted with a compound of formula (II) is a chlorinated aliphatic solvent such as methylene chloride,
– the organic solvent in which the paratoluene sulphonic acid is reacted with a compound of formula (III) is an aromatic solvent such as benzene, toluene or xylene, the functional derivative of the acid of formula (IV) is the acid chloride and the acid chloride is reacted with the alcohol R–OH in the presence of a tertiary base,
– the acid of formula (IV) is reacted with the alcohol ROH, in the presence of dicyclohexyl carbodiimide or diisopropyl carbodiimide and dimethyl amino pyridine.

3. Preparation process according to claim 1, characterised in that the compound of formula (II):

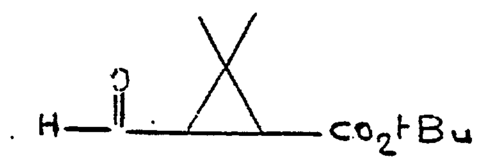

(II)

is reacted with tetraiodomethane, triphenylphosphine and zinc in an organic solvent so as to obtain a compound of formula (IV):

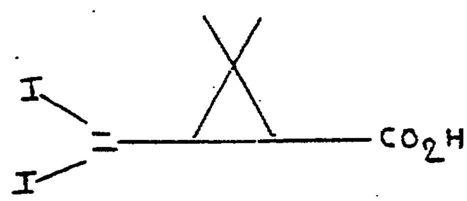

(IV)

then the synthesis is completed as in claim 1.

4. Preparation process according to claim 3, characterised in that the organic solvent used is a chlorinated aliphatic solvent such as methylene chloride.

5. Process according to any one of claims 1 to 4, characterised in that to start with an alcohol of formula R–OH or a functional derivative of this alcohol is used, formula in which R represents:
– either a linear or branched alkyl radical, containing from 1 to 8 carbon atoms,
– or a benzyl radical,
– or a group of formula:

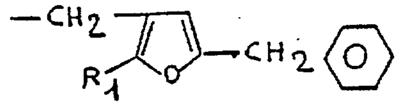

in which $R_1$ represents a hydrogen atom or a methyl radical, or a group of formula:

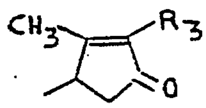

in which $R_3$ represents an organic aliphatic radical containing from 2 to 6 carbon atoms and one or more carbon-carbon unsaturations and more particularly the radicals
$-CH_2=CH=CH_2$, $-CH_2-CH=CH-CH_3$,
$-CH_2-CH=CH-C_2H_5$, $-CH_2-CH=CH-CH=CH_2$, $CH_2-C\equiv CH$,
– or a group of formula:

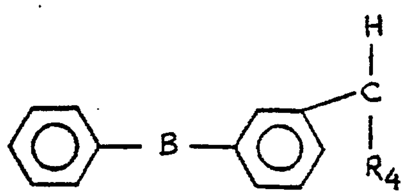

in which B represents a C = O group or an oxygen atom and $R_4$ represents a hydrogen atom, a methyl radical, a $-C\equiv N$ group, a $-C\equiv CH$ group, a 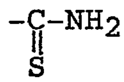 group,
– or a group of formula:

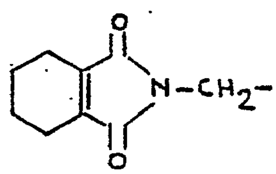

– or a group of formula:

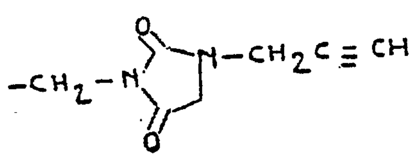

– or a group of formula:

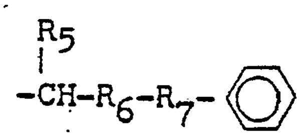

in which $R_5$ represents a hydrogen atom or a –CN radical, $R_6$ represents a thiazolyl radical in which the 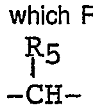 bond can be at any one of the available positions, $R_7$ represents a $-CH_2-$ radical or an oxygen atom:

– or a group of formula:

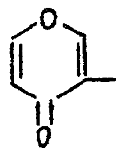

– or a group of formula:

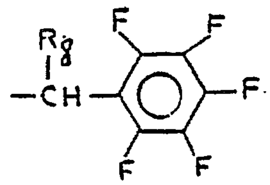

in which $R_8$ represents a hydrogen atom or a CN radical,
– or a group of formula:

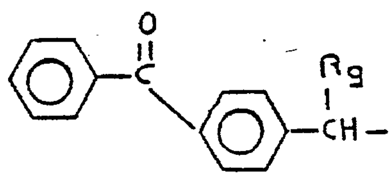

in which $R_9$ represents a hydrogen atom or a –CN radical,
– or a group of formula:

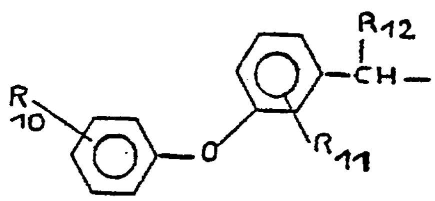

in which $R_{12}$ represents a hydrogen atom, a methyl, ethynyl or cyano radical and $R_{10}$ and $R_{11}$ different from one another represent a hydrogen, fluorine, chlorine or bromine atom,
– or a group of formula:

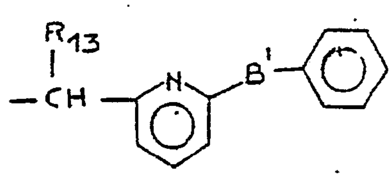

in which $R_{13}$ represents a hydrogen atom, a methyl, ethynyl or cyano radical and B′ represents an oxygen or sulphur atom,
– or a group of formula:

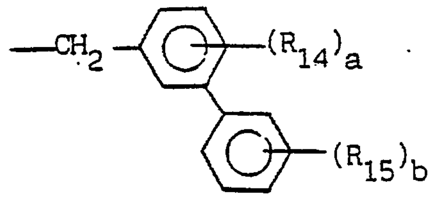

in which $R_{14}$ represents a halogen atom, an alkyl radical containing from 1 to 6 carbon atoms or a trifluoromethyl radical, $R_{15}$ represents a halogen atom, an alkyl radical containing from 1 to 6 carbon atoms, an alkoxy radical containing from 1 to 6 carbon atoms or a trifluoromethyl radical, a is 0, 1, 2, 3 or 4 and b is 0, 1, 2, 3, 4, or 5.

6. Process according to any one of claims 1 to 4, characterised in that to start with an alcohol of formula R–OH or a functional derivative of this alcohol is used, formula in which R represents a group of formula:

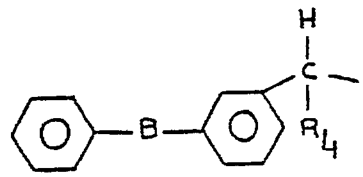

in which B represents a C = O group or oxygen and $R_4$ represents hydrogen, a methyl radical, a C ≡ N group, a –C ≡ CH group or a 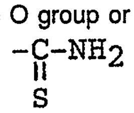 group.

7. Process according to any one of claims 1 to 4, characterised in that to start with an alcohol of formula R–OH or a functional derivative of this alcohol is used, formula in which R represents a group of formula:

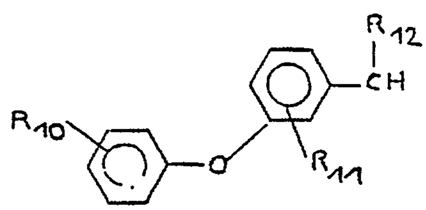

in which $R_{12}$ represents a hydrogen atom, a methyl, ethynyl or cyano radical, and $R_{10}$ and $R_{11}$ different to one another represent hydrogen or a fluorine, chlorine or bromine atom.

8. Process according to any one of claims 1 to 4, characterised in that to start with an alcohol of formula R–OH or a functional derivative of this alcohol is used, formula in which R represents a group of formula:

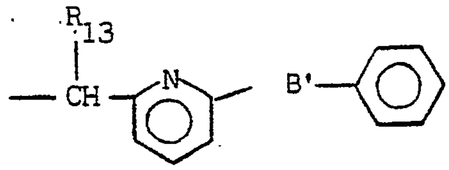

in which $R_{13}$ represents a hydrogen atom, a methyl, ethynyl or cyano radical and B′ represents an oxygen or sulphur atom.

9. Process according to any one of claims 1 to 4, characterised in that to start with an alcohol of formula R–OH or a functional derivative of this alcohol is used, formula in which R represents a group of formula:

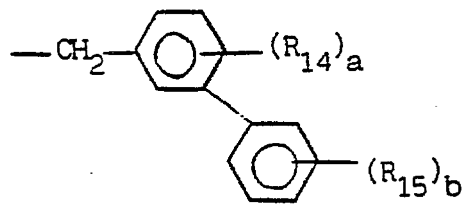

in which $R_{14}$ represents a halogen atom, an alkyl radical containing from 1 to 6 carbon atoms or a trifluoromethyl radical, $R_{15}$ represents a halogen atom, an alkyl radical containing from 1 to 6 carbon atoms, an alkoxy radical containing from 1 to 6 carbon atoms or a trifluoromethyl radical, a is 0, 1, 2, 3 or 4 and b is 0, 1, 2, 3, 4 or 5.

10. Process according to any one of claims 1 to 4, characterised in that to start with an alcohol of formula R–OH or a functional derivative of this alcohol is used, formula in which R represents a group of formula:

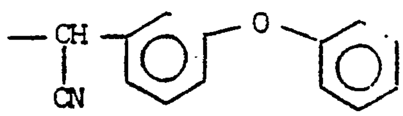

,

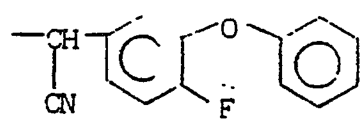

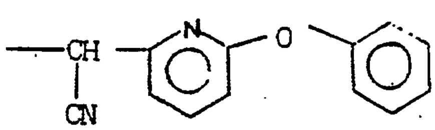

or

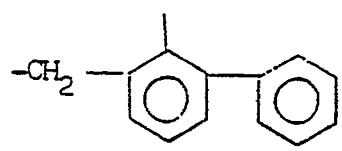

11. Process according to any one of claims 1 to 4, characterised in that to start with a derivative of formula (II) is used in which the configuration is 1R, cis or 1RS, cis and an alcohol of formula R–OH, or a functional derivative of this alcohol, formula in which R represents a group of formula:

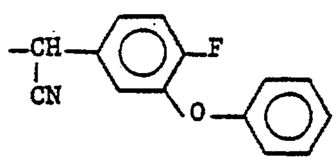

of configuration (S) or (R, S).